# EUROPÄISCHE PATENTSCHRIFT

(11) **EP 3 551 104 B1**
(45) Veröffentlichungstag und Bekanntmachung des Hinweises auf die Patenterteilung: **13.01.2021**
(21) Anmeldenummer: 17825377.9
(22) Anmeldetag: 28.11.2017
(51) Int. Cl.: A61B 17/70

(54) **CHIRURGISCHES REPOSITIONSINSTRUMENT**
SURGICAL REPOSITIONING INSTRUMENT
INSTRUMENT DE REPOSITIONNEMENT CHIRURGICAL

(30) Priorität: 08.12.2016 DE 102016224503
(43) Veröffentlichungstag der Anmeldung: 16.10.2019
(73) Patentinhaber: Premiere Medical GmbH, 89155 Erbach (DE)
(72) Erfinder: VAZIFEHDAN, Dr. Farzam, 70195 Stuttgart (DE); PIPPAN, Dr. Mathias, 55122 Mainz (DE); REITH, Dr. Michael, 55130 Mainz (DE); NILSSON, C. Michael, Moreland Hills, Ohio 44022 (US); SCHÖNHÖFFER, Dr. Helmut, 89155 Erbach (DE)
(74) Vertreter: Kohler Schmid Möbus Patentanwälte
(86) Internationale Anmeldenummer: PCT/EP2017/080680
(87) Internationale Veröffentlichungsnummer: WO 2018/104112

(56) Entgegenhaltungen:
- US-A1- 2009 228 053
- US-A1- 2010 324 609
- US-A1- 2012 191 144
- US-A1- 2012 215 266
- US-A1- 2012 283 786

## Beschreibung

Die Erfindung betrifft ein chirurgisches Repositionsinstrument zum Absenken und Fixieren eines Fixierstabs im Tulpenkopf einer Knochenschraube.

Bei der Instrumentierung der Wirbelsäule werden in der klinischen Praxis Implantatsysteme mit Knochenschrauben eingesetzt, die über, zumeist biege- und torsionssteife, Fixierstäbe untereinander verbunden werden. Die Knochenschrauben dieser sogenannten Stab-Schraubensysteme weisen einen Gewindeschaft mit einem sogenannten Tulpenkopf auf, der eine Fixierstabaufnahme aufweist. Zum Absenken und Fixieren des Fixierstabs in der Fixierstabaufnahme eines solchen Tulpenkopfs werden sogenannte Repositionsinstrumente eingesetzt. Die am Markt verfügbaren Repositionsinstrumente sind häufig nur schwer handzuhaben, so dass, insbesondere bei minimalinvasiven operativen Zugangswegen, unnötige Gewebstraumatisierungen mitunter nicht zu vermeiden sind. Der Heilungsverlauf kann dadurch verzögert werden. Darüber hinaus ist eine ordnungsgemäße hygienische Aufbereitung, d. h. Reinigung und Desinfektion bzw. Sterilisation, der am Markt verfügbaren Repositionsinstrumente häufig nur mit einem hohen Zeit- und Kotenaufwand möglich.

Gattungsgemäße Repositionsinstrumente sind aus US 2012/215266 A1, US 2010/324609 A1, US 2012/191144 A1 und US 2012/283786 A1 bekannt.

Es ist deshalb die Aufgabe der Erfindung, ein Repositionsinstrument anzugeben, das eine vereinfachte und dabei zugleich sichere Handhabung aufweist und das vorzugsweise vereinfacht hygienisch aufzubereiten ist.

Das erfindungsgemäße Repositionsinstrument weist die in Anspruch 1 angegebenen Merkmale auf. Weiterbildungen der Erfindung sind in den Unteransprüchen sowie in der Beschreibung angegeben.

Das chirurgische Repositionsinstrument zeichnet sich durch einen besonders einfachen konstruktiven Aufbau und eine vereinfachte Handhabung aus. Das Repositionsinstrument kann mit seinen zumindest zwei Rastarmen der Koppelhülse besonders gewebeschonend auf dem Tulpenkopf einer jeweiligen Knochenschraube, die zuvor im Knochengewebe des Patienten verankert wurde, in Richtung der Längsachse des Repositionsinstruments aufgesteckt und mit dem Tulpenkopf verrastet werden. Die Rastarme werden dabei allein durch deren Kontakt mit dem Tulpenkopf relativ zur Längsachse des Repositionsinstruments nur soweit radial nach außen deflektiert bzw. ausgelenkt, dass der Tulpenkopf in axialer Richtung zwischen die zumindest zwei Rastarme gleiten und mit diesen verrasten kann. Für ein vereinfachtes Heranführen und Ankoppeln des Repositionsinstruments an den im Operationssitus angeordneten Tulpenkopf kann zusätzlich ein Führungsstab eingesetzt werden, der in das Tulpengehäuse eingeführt und an diesem, vorzugsweise zugfest, lösbar verkoppelt wird. Dies kann beispielsweise durch ein Einschrauben des Führungsstabs in das üblicherweise vorhandene Innengewinde der Wandschenkel des Tulpenkopfs oder auch durch ein Klemmen bzw. Verrasten des Führungsstabs im Tulpenkopf erreicht werden.

Das Repositionsinstrument kann dann mit dem hülsenförmigen Stabdrücker auf den Führungsstab aufgefädelt und in axialer Richtung entlang des Führungsstabs bis zum Tulpenkopf vorgeschoben werden. Die Arretierhülse des Repositionsinstruments kann vom Benutzer (Operateur) des Repositionsinstruments auf einfache und bequeme Weise unabhängig vom Stabdrücker bzw. dessen Betätigung - mithin ohne eine damit einhergehende sogenannte Reduzierung, d. h. Absenkung, des Fixierstabs mittels des Stabdrückers - aus ihrer Freigabeposition in ihre Arretierposition verschoben werden. In der Arretierposition übergreift die Arretierhülse die Rastarme der Koppelhülse außenseitig in einer radialen Richtung, so dass diese in ihrer Ankopplungs- bzw. verrasteten Position am Tulpengehäuse der Knochenschraube gesichert ist.

Bei dem erfindungsgemäßen Repositionsinstrument sind die Arretierhülse und der Stabdrücker somit voneinander mechanisch entkoppelt. Die beiden Bauteile sind mit anderen Worten mechanisch betrachtet nicht miteinander bewegungsgekoppelt verstellbar. Dadurch wird die Handhabung und die Kontrollierbarkeit des Repositionsinstruments wesentlich vereinfacht. Darüber hinaus können dadurch unerwünschte Bewegungen des Repositionsinstruments im Operationssitus weiter reduziert werden. Das Risiko einer Gewebstraumatisierung kann dadurch insgesamt nochmals weiter verringert werden. Darüber hinaus kann dadurch die Bediensicherheit des Repositionsinstruments verbessert werden. Auch kann der konstruktive Aufbau des Repositionsinstruments einfach gehalten werden, was für die Herstellung, Wartung und die hygienische Aufbereitung des Repositionsinstruments Vorteile bietet.

Das Griffteil erlaubt ein bequemes und sicheres Halten des Repositionsinstruments und ist in seiner Formgebung bevorzugt auf die Anatomie der menschlichen Hand abgestimmt. Der am Griffteil schwenkbar gelagerte Betätigungshebel dient dem Vorschieben des Stabdrückers relativ zur Koppelhülse, d.h. dem (vollständigen) Absenken des Fixierstabs in den Tulpenkopf der Knochenschraube. Der Betätigungshebel ist vorzugsweise um eine zur Längsachse orthogonal verlaufend angeordnete Schwenkachse verschwenkbar. Dadurch kann der Betätigungshebel bequem mit den Fingern der das Repositionsinstrument haltenden Hand des Benutzers bedient werden. Es versteht sich, dass der Kraftarm dabei funktionell betrachtet länger gewählt ist, als der Lastarm, um eine kraftsparende Handhabung und damit eine überragende Kontrolle des Repositionsinstruments zu gewährleisten. Der Stabdrücker ist vorzugsweise als ein Hohlprofil ausgeführt, sodass der eingangs genannte Fixierstab im Tulpenkopf der Knochenschraube mittels einer Fixierschraube und eines in den Stabdrücker eingeführten Drehwerkzeugs festgesetzt werden kann.

Ein nochmals höheres Maß an Bediensicherheit des Repositionsinstruments kann nach der Erfindung dadurch erreicht werden, dass die Arretierhülse in ihrer Arretierposition relativ zur Koppelhülse arretierbar, insbesondere mit dem Griffteil verrastbar, ist. Ein unbeabsichtigtes Lösen der Rastarme der Arretierhülse kann dadurch zuverlässig unterbunden werden.

Nach einer besonders bevorzugten Weiterbildung der Erfindung ist der Lastarm des Betätigungshebels über einen im Griffteil längsverschieblich gelagerten Mitnehmerschlitten mit dem Stabdrücker verkoppelt. In diesem Fall greift der Lastarm somit über den Mitnehmerschlitten am Stabdrücker an. Durch den Einsatz eines solchen Mitnehmerschlittens kann die Schwenkbewegung des Betätigungsgriffs auf konstruktiv einfache und wenig störanfällige Weise in eine translatorische Bewegung des Stabdrückers relativ zur Koppelhülse umgesetzt werden. Darüber hinaus kann das Griffteil des Repositionsinstruments kompakt ausgeführt werden und zugleich für den Mitnehmerschlitten als eine schützende Umhausung fungieren.

Der Mitnehmerschlitten besteht im Hinblick auf dessen Lastaufnahmevermögen vorzugsweise aus Metall. Zur mechanischen Verbinden des Mitnehmerschlittens mit dem Betätigungshebel kann der Mitnehmerschlitten Lagerzapfen aufweisen, die jeweils in ein Langloch des Betätigungshebels, speziell des Lastarms, eingreifen. Die Lagerzapfen können sich durch axial verlaufende (Führungs-) Schlitze des Griffteils nach außen erstrecken. Dadurch kann eine besonders präzise und störungsarme Führung des Mitnehmerschlittens erreicht werden.

Nach der Erfindung weist das Griffteil vorzugsweise ein Seitenwandelement mit einer, vorzugsweise T-förmigen, Führungs- bzw. Längsnut auf, in der der Mitnehmerschlitten geführt ist. Durch diese Nutführung kann einer Fehlfunktion des Mitnehmerschlittens, beispielsweise durch dessen Verkanten im Griffteil, entgegengewirkt werden. Auch kann das Seitenwandelement als ein Anbauteil des Griffteils mit geringen Fertigungstoleranzen, d. h. als Präzisionsbauteil, kostengünstig bereitgestellt werden. Im Falle der T-förmig ausgeführten Nut kann eine besonders zuverlässige Mehrpunkt- bzw. Mehrlinienführung des Mitnehmerschlittens realisiert werden.

Das Seitenwandelement kann erfindungsgemäß am Griffteil schwenkbar gelagert sein. Dadurch kann einerseits der Funktionszustand des Repositionsinstruments im Bereich des Mitnehmerschlittens visuell geprüft werden. Ist der Mitnehmerschlitten in einer T-förmigen Führungsnut des Seitenwandelements geführt bzw. verschiebbar gelagert, so kann das Seitenwandelement eine Doppelfunktion aufweisen. So kann der Mitnehmerschlitten durch das Herausschwenken des Seitenwandelements aus dem Griffteil der Mitnehmerschlitten außer Eingriff mit dem Stabdrücker gebracht werden. Dies erlaubt ein zügiges Zerlegen des Repositionsinstruments für dessen hygienische Aufbereitung, d. h. Reinigung bzw. Desinfektion.

Nach einer alternativen Weiterbildung der Erfindung ist der Stabdrücker mit einem oder mehreren seitlichen Profilfortsätzen versehen, der/die sich außenseitig vom Stabdrücker in einer radialen Richtung wegerstreckt bzw. wegerstrecken. Diese Profilfortsätze können mit dem Lastarm des Betätigungshebels unmittelbar verkoppelt sein.

Nach einer Weiterbildung der Erfindung weisen die Arretierhülse und die Koppelhülse jeweils eine seitliche Wandöffnung auf, die im betriebsbereiten Zustand des Repositionsinstruments in radialer Richtung zumindest teilweise zueinander fluchtend angeordnet sind, wobei sich der Mitnehmerschlitten durch die beiden Wandöffnungen dieser Hülsen in radialer Richtung hindurcherstreckt. Dadurch kann das Repositionsinstrument besonders kompakt und mit einer geringen Anzahl erforderlicher Teile ausgeführt werden.

Im Hinblick auf eine nochmals weiter verbesserte Handhabung des Repositionsinstruments ist der Betätigungshebel vorzugsweise gegen die Kraft eines Federelements, insbesondere eines Torsionsfederelements, aus seiner Ruhestellung auslenkbar bzw. herausbewegbar.

Für eine einfache Montage und Demontage des Repositionsinstruments kann die Koppelhülse zwei, vorzugsweise federelastisch angelenkte, Radialvorsprünge aufweisen, die sich im betriebsbereiten Zustand des Repositionsinstruments jeweils in radialer Richtung durch eine langlochförmige Ausnehmung der Arretierhülse hindurch in eine Lagerausnehmung des Griffteils hineinerstrecken. Dadurch kann eine (lösbare) drehfeste Arretierung der Arretierhülse wie auch der Koppelhülse am Griffteil auf einfache und kostengünstige Weise erreicht werden. Darüber hinaus kann die Koppelhülse am Griffteil in axialer Richtung (lösbar) lagefixiert werden. Die Radialvorsprünge sind vorzugsweise an Federarmen der Arretierhülse angeformt, die mit der Arretierhülse einstückig ausgebildet sind. Diese bietet bei der Herstellung, Montage und Aufbereitung des Repositionsinstruments Vorteile.

Das Griffteil kann nach der Erfindung einenends eine öffenbare Lagerschelle aufweisen, die vorzugsweise aus Metall besteht. Die Lagerschelle kann die Lagerausnehmungen für die Radialvorsprünge der Koppelhülse umfassen bzw. bilden. Besteht die Lagerschelle aus Metall, so kann ein hohes mechanisches Lastaufnahmevermögen des Griffteils, insbesondere im Bereich seines Kopfabschnitts, gewährleistet werden. Darüber hinaus kann mittels einer solchen Lagerschelle ein unbeabsichtigtes Entfernen der Arretierhülse sowie auch der Koppelhülse aus dem Griffteil in axialer Richtung verhindert werden.

Der Stabdrücker ist nach der Erfindung vorzugsweise mittels einer Rasteinrichtung in zumindest einer axialen Verschiebeposition, vorzugsweise in unterschiedlichen axialen (Vorschub-)Positionen, relativ zur Koppelhülse verrastbar bzw. arretierbar. Im erstgenannten Fall ist der Stabdrücker bevorzugt in seiner distalen Absenkposition gegenüber der Koppelhülse axial lagefixierbar. Im letztgenannten Fall kann der Stabdrücker an variablen axialen Verschiebepositionen relativ zur Koppelhülse bzw. dem Tulpengehäuse gesichert werden. Dies ermöglicht einen besonders kraftsparenden und vorsichtigen Betriebseinsatz des Repositionsinstruments. Nach dem vollständigen Absenken des Fixierstabs im Tulpengehäuse der Knochenschraube kann das Repositionsinstrument vom Benutzer losgelassen werden, so dass dieser den Fixierstab, beispielsweise mittels einer in den Tulpenkopf einzudrehenden Schraube, dauerhaft im Tulpenkopf fixieren kann, ohne dabei das Repositionsinstrument halten zu müssen.

Die Rasteinrichtung umfasst nach einer besonders bevorzugten Weiterbildung ein Rastelement das im Griffteil, vorzugsweise verschiebbar, gelagert ist und das mit einem Rastprofil des Stabdrückers zusammenwirkt. Unter konstruktiven Gesichtspunkten hat es sich dabei als besonders vorteilhaft erwiesen, wenn das Rastelement die Arretierhülse, die Koppelhülse und den Stabdrücker im betriebsbereiten Zustand des Repositionsinstruments ringförmig umgreift. Dadurch kann das Rastelement einerseits unverlierbar im Griffteil angeordnet sein. Andererseits kann dadurch eine besonders großflächige axiale Anlage des Rastelements an dem Stabdrücker realisiert werden. Dies bietet ein hohes Lastaufnahmevermögen der Rastverbindung zwischen dem Griffteil und dem Stabdrücker.

Für einen besonders einfachen, sicheren und zugleich wenig störanfälligen Betriebseinsatz des Repositionsinstruments ist das Rastelement vorzugsweise durch die Kraft eines Federelements radial in Richtung seiner Raststellung am Stabdrücker vorgespannt angeordnet. Dadurch kann eine selbstverrastende Funktion der Rasteinrichtung erreicht werden. Auch kann dadurch einer benutzerseitigen Fehlbedienung des Repositionsinstruments zuverlässig entgegengewirkt werden.

Nach einer besonders bevorzugten Weiterbildung der Erfindung ist die Arretierhülse mittels des Rastelements in ihrer Arretierposition relativ zur Koppelhülse in axialer Richtung arretierbar. Das Rastelement weist in diesem Fall somit eine Doppelfunktion auf, wodurch die Anzahl der erforderlichen Bauteile des Repositionsinstruments weiter minimiert werden kann. Dies bietet bei der Herstellung, der Montage und Wartung sowie auch bei der hygienischen Aufbereitung des Instruments Vorteile.

Besonders bevorzugt erstreckt sich das Rastelement in Arretierposition der Arretierhülse abschnittsweise über die vorstehend erläuterte Wandöffnung der Arretierhülse in radialer Richtung in die Arretierhülse hinein und liegt in axialer Richtung an einem unteren Rand der Wandöffnung des Arretierelements an. Dadurch kann ein unbeabsichtigtes axiales (Rück-)Verschieben der Arretierhülse aus ihrer (einmal erreichten) Arretierstellung zuverlässig vermieden werden. Es versteht sich, dass das Rastelement diesbezüglich ohne oder mit einem nur geringen axialen Spiel im Griffteil gelagert sein muss. Zur Lagerung des Rastelements kann das Griffteil insbesondere ein seitlich offenes Aufnahmefach aufweisen. Das Aufnahmefach weist mit anderen Worten eine Seitenwandöffnung auf.

Die Koppelhülse kann an ihrem distalen freien Endabschnitt einen Führungsarm für den Tulpenkopf aufweisen, der sich in axialer Richtung zwischen den beiden Rastarmen erstreckt und von diesen jeweils unter Ausbildung eines Axialspalts beabstandet ist. Der Führungsarm ist dabei im Vergleich zu den Rastarmen vorzugsweise biegesteif, d.h. in radialer Richtung formstabil, ausgebildet. Dadurch kann das Repositionsinstrument nochmals einfacher auf den Tulpenkopf der Knochenschraube aufgesteckt und nochmals zuverlässiger an diesem gegenüber einem unerwünschten Abrutschen vom Tulpenkopf gesichert werden. Die beiden Rastarme und der Führungsarm erstrecken sich vorzugsweise über die Hälfte oder nahezu die Hälfte des Gesamtumfangs der Koppelhülse.

Die Koppelhülse kann nach einer bevorzugten Ausführungsform der Erfindung zwei weitere Rastarme aufweisen, zwischen denen sich in axialer Richtung vorzugsweise ein weiterer Führungsarm erstreckt. Dadurch kann der Tulpenkopf umfangsseitig an weiteren Positionen gegriffen werden, wodurch eine mechanisch besonders belastbare Verkopplung des Repositionsinstruments mit dem Tulpenkopf der Knochenschraube erreicht werden kann.

Die Arretierhülse weist an ihrem distalen freien Endabschnitt vorzugsweise Führungszungen auf, die jeweils in eine dazu korrespondierende Längsnut der Koppelhülse eingreifen, wobei sich die Längsnuten jeweils zumindest abschnittsweise an einer Seitenflanke eines der Rastarme angeordnet sind. Dadurch können die Rastarme der Koppelhülse durch die in ihrer Arretierposition angeordnete Arretierhülse nochmals besser vor einem unerwünschten seitlichen Abrutschen vom Tulpenkopf gesichert werden.

Die Längsnuten der Koppelhülse können sich nach der Erfindung jeweils bis zum proximalen Ende der Koppelhülse erstrecken. Dadurch kann die Arretierhülse auf der Koppelhülse vereinfacht montiert und die beiden Bauteile zugleich drehfest miteinander verbunden werden. Dies ermöglicht eine besonders zuverlässige Funktion des Repositionsinstruments sowie ein hohes Maß an Bediensicherheit.

Nach einer bevorzugten Weiterbildung der Erfindung kann die Arretierhülse gegen die Kraft eines, vorzugsweise innerhalb der Arretierhülse angeordneten, Federelements aus seiner Freigabeposition in die Arretierposition verschiebbar sein. Nach einem Entriegeln des vorstehend genannten Rastelements kann die Arretierhülse dadurch selbsttätig (durch Federkraft) auf der Koppelhülse in ihre Ausgangsstellung bzw. in ihre Ruheposition zurückgleiten.

Im Hinblick auf eine möglichst geringe Masse des Repositionsinstruments kann das Griffteil einen Grundkörper aufweisen, der aus Kunststoff oder einem Kunststoffverbundmaterial besteht. Lastaufnehmende Teile des Repositionsinstruments können aus einem Metall bestehen. Insoweit können insbesondere der Kopf- bzw. der Fußabschnitt des Griffteils teilweise oder vollständig aus Metall bestehen.

Es versteht sich, dass das Repositionsinstrument vorzugsweise insgesamt aus autoklavierbaren Materialien besteht.

Weitere Vorteile der Erfindung ergeben sich aus der Beschreibung und der Zeichnung. Die gezeigten und beschriebenen Ausführungsformen sind nicht als abschließende Aufzählung zu verstehen, sondern haben vielmehr beispielhaften Charakter für die Schilderung der Erfindung.

In der Zeichnung zeigen
- Fig. 1: ein Repositionsinstrument zum Absenken und Fixieren eines Fixierstabs im Tulpenkopf einer Knochenschraube, mit einem Griffteil und mit mehreren Hülsen, die zur Längsachse koaxial verlaufend angeordnet sind und sich in das Griffteil hineinerstrecken, in einer ersten perspektivischen Ansicht;
- Fig. 2: das Repositionsinstrument gemäß Figur 1 in einer weiteren perspektivischen Ansicht;
- Fig. 3: das Repositionsinstrument gemäß Fig. 1 in einem Längsschnitt;
- Fig. 4: einen vergrößert dargestellten Detailausschnitt des Repositionsinstruments gemäß Fig. 3;
- Fig. 5: das Griffteil des Repositionsinstruments gemäß Fig. 1 mit einer explodierten Darstellung seiner Teile, in einer perspektivischen Ansicht;
- Fig. 6: eine Arretierhülse des Repositionsinstruments gemäß Fig. 1 in einer freigestellten perspektivischen Ansicht;
- Fig. 7: eine Koppelhülse des Repositionsinstruments gemäß Fig. 1 mit zwei distalen Rastarmen, in einer freigestellten perspektivischen Ansicht;
- Fig. 8: einen Hohlprofil-Stabdrücker des Repositionsinstruments gemäß Fig. 1 in einer freigestellten perspektivischen Ansicht;
- Fig. 9: eine Detailansicht des Repositionsinstruments gemäß Fig. 1 mit einem Tulpenkopf einer Knochenschraube, in einer perspektivischen Ansicht;
- Fig. 10: eine Detailansicht des Repositionsinstruments gemäß Fig. 1, das am Tulpenkopf einer Knochenschraube angekoppelt und arretiert ist, in einer perspektivischen Ansicht;
- Fig. 11: das Repositionsinstrument gemäß Fig. 1 im an einem Tulpenkopf angekoppelten und arretierten Zustand, vor dem Niederdrücken des Stabdrückers;
- Fig. 12: das Repositionsinstrument gemäß Fig. 11 in einem freigestellten Längsschnitt;
- Fig. 13: das Repositionsinstrument gemäß Fig. 12 in einer ausschnittsweisen Detailvergrößerung;
- Fig. 14: das Repositionsinstrument gemäß Fig. 11 mit einem in das Tulpengehäuse abzusenkenden Fixierstab, in einer perspektivischen Ansicht;
- Fig. 15: das Repositionsinstrument gemäß Fig. 11 nach dem Niederdrücken des Stabdrückers, in einer perspektivischen Ansicht;
- Fig. 16: eine ausschnittsweise vergrößerte Detaildarstellung des am Tulpenkopf angekoppelten und arretierten Repositionsinstruments gemäß Fig. 11 mit einem im Tulpengehäuse vollständig abgesenkten Fixierstab, in einer perspektivischen Darstellung;
- Fig. 17: das Repositionsinstrument gemäß Fig. 15 in einem Längsschnitt;
- Fig. 18: eine ausschnittsweise Detailvergrößerung des Repositionsinstruments gemäß Fig. 17;
- Fig. 19: eine alternative Ausführungsform einer Koppelhülse des Repositionsinstruments gemäß Fig. 1, in einer perspektivischen Darstellung;
- Fig. 20: eine alternative Ausführungsform einer Arretierhülse des Repositionsinstruments gemäß Fig. 1 für eine Koppelhülse gemäß Fig. 19, in einer perspektivischen Darstellung;
- Fig. 21: das Repositionsinstrument gemäß Fig. 1 im entriegelten Zustand für das Zerlegen des Repositionsinstruments zwecks Aufbereitung desselben, in einer Schnittdarstellung;
- Fig. 22: ein Blockdiagramm eines erfindungsgemäßen Verfahrens zum Montieren des Repositionsinstruments gemäß Fig. 1;

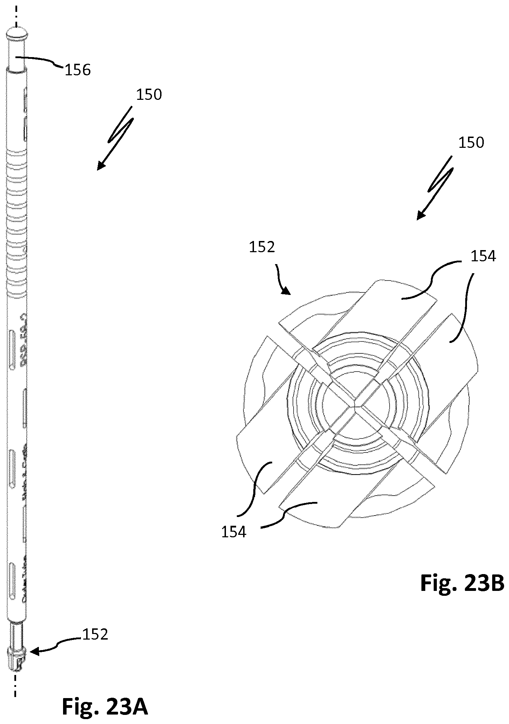

- Fig. 23A: einen Führungsstab für ein Repositionsinstrument gemäß Fig. 1, der über einen Kopplungsabschnitt mit dem Tulpenkopf einer Knochenschraube mechanisch verkoppelbar ist; und
- Fig. 23B: der Kopplungsabschnitt des Führungsstabs in einer stirnseitigen Ansicht.

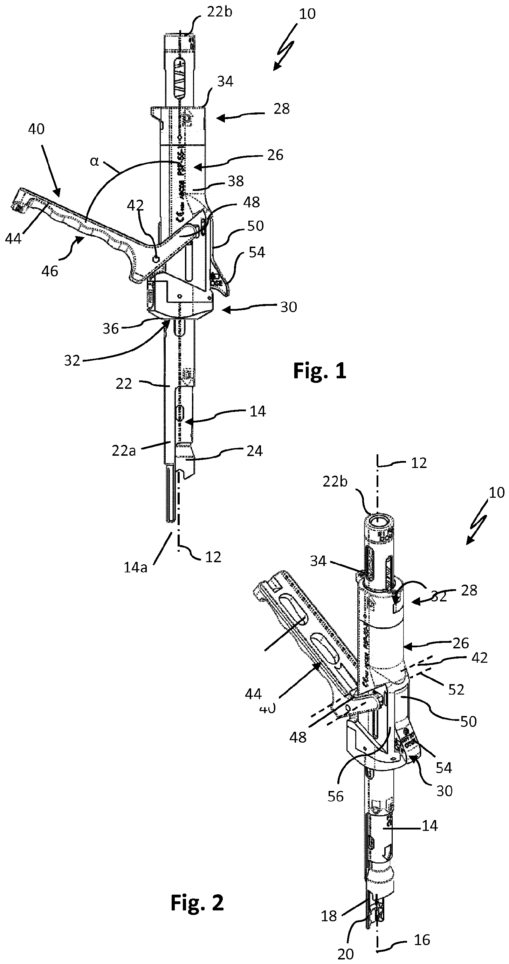

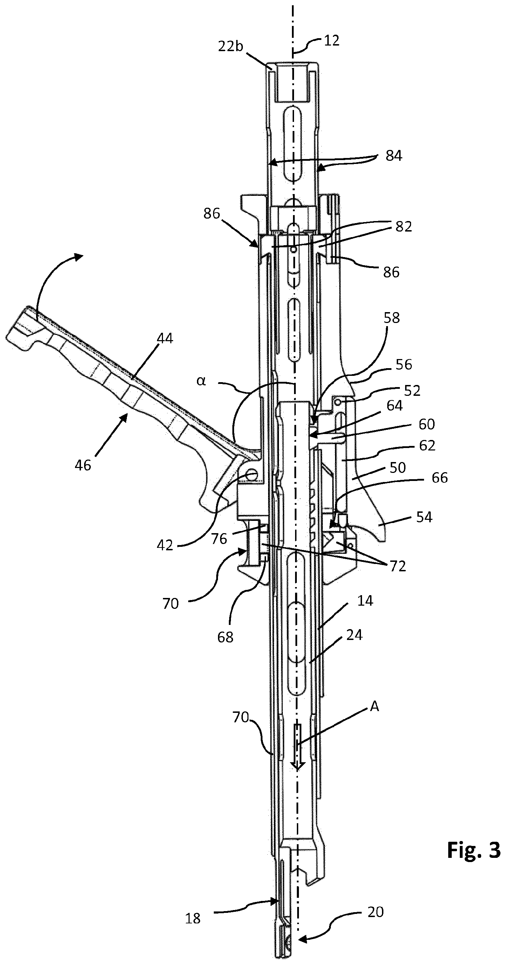

In den **Figuren 1** und **3** ist ein insgesamt mit **10** bezeichnetes chirurgisches Repositionsinstrument für die Wirbelsäulenchirurgie in einer perspektivischen Ansicht gezeigt. Das Repositionsinstrument 10 dient dazu, einen sogenannten Fixierstab im Tulpenkopf einer Knochenschraube (in den Figuren 1 bis 3 nicht gezeigt) abzusenken und im Tulpenkopf mittels einer Schraube oder dergleichen zu fixieren. Derlei Repositionsinstrumente 10 werden auch als sogenannte "Persuador" bezeichnet.

Das Repositionsinstrument 10 ist in den Figuren 1 bis 2 in seinem funktionsfähigen Betriebszustand gezeigt. Das Repositionsinstrument 10 umfasst eine Längsachse **12** und drei zumindest abschnittsweise hülsenförmige Bauteile, die auf der Längsachse des Repositionsinstruments 10 koaxial angeordnet sind.

Zum Ankoppeln des Repositionsinstruments 10 am Tulpenkopf der Knochenschraube dient eine Koppelhülse **14.** Die Koppelhülse 14 weist eine Längsachse **16** auf, die mit der Längsachse 12 des Repositionsinstruments 10 zusammenfällt. Die Koppelhülse 14 weist einen distalen Endabschnitt **14a** mit zwei Rastarmen **18** auf. Die Rastarme 18 sind in einer zur Längsachse 16 der Koppelhülse 14 bzw. zur Längsachse 12 des Repositionsinstruments radialen Richtung federelastisch auslenkbar, so dass das Repositionsinstrument 10 über die Rastarme 18 auf das Tulpengehäuse der Knochenschraube aufsteckbar und mit diesem verrastbar ist. Ein freier (distaler) Endabschnitt der Rastarme 18 ist mit **20** bezeichnet.

Auf der Koppelhülse 14 ist eine Sperr- oder Arretierhülse **22** angeordnet, die zwischen einer in den Figuren 1 bis 3 gezeigten proximalen Freigabeposition und einer distalen Arretierposition längsverschiebbar angeordnet ist. Durch die in Freigabeposition angeordnete Arretierhülse 22 ist die radiale Auslenkung der Rastarme 18 der Arretierhülse ermöglicht, während eine solche bei in Arretierposition angeordneter Arretierhülse 22 gehemmt bzw. gesperrt ist.

Zum Kontaktieren und Absenken des Fixierstabs dient ein Druckglied oder Stabdrücker **24.** Der Stabdrücker 24 ist innerhalb der Koppelhülse zwischen einer (proximalen) Ruhe- oder Ausgangsposition und einer (distalen) Absenkposition relativ zur Koppelhülse 14 in axialer Richtung verschiebbar angeordnet.

Das Repositionsinstrument 10 weist ferner ein Griffteil **26** mit einem Kopfabschnitt **28** und einem Fußabschnitt **30** auf. Das Griffteil 26 ist mit einer längsaxialen Durchgangsausnehmung **32** versehen. Die Durchgangsausnehmung 32 erstreckt sich hier vom Kopfabschnitt 28 bis zum Fußabschnitt 30 bzw. vom proximalen Ende **34** des Griffteils 26 bis zum distalen Ende **36** des Griffteils 26. In die Durchgangsausnehmung 32 erstrecken sich die radial äußere Arretierhülse 22, der in radialer Richtung innenliegend angeordnete Stabdrücker 24 sowie auch die zwischen der Arretierhülse 22 und dem Stabdrücker 24 angeordnete Koppelhülse 14 hinein. Die Arretierhülse 22 steht mit ihrem proximalen Ende **22b** in axialer Richtung über das Griffteil 26 hervor. Mit anderen Worten überragt die Arretierhülse 22 das Griffteil 26 in proximaler Richtung.

Das Griffteil 26 umfasst hier einen Grundkörper **38,** der aus Gewichtsgründen vorzugsweise aus Kunststoff oder einem Kunststoffverbundmaterial besteht. Am Griffteil 26 bzw. am Grundkörper 38 ist ein Betätigungshebel **40** für den Stabdrücker um eine erste Schwenkachse **42** schwenkbar gelagert. Die erste Schwenkachse ist zur Längsachse 12 orthogonal verlaufend ausgerichtet. Der Betätigungshebel 40 weist einen Kraftarm **44** mit einer vorzugswiese ergonomisch ausgeformten Fingeraufnahme **46** für den Benutzer des Repositionsinstruments auf. Der Kraftarm 44 ist manuell aus seiner in Fig. 1 gezeigten Neutralstellung, in der der Kraftarm 44 unter einem spitzen Winkel **α** zur Längsachse 12 des Repositionsinstruments 10 vom Griffteil 26 seitlich weg steht, um die erste Schwenkachse 42 an das Griffteil 26 heranbewegbar, um den Stabdrücker 24 in axialer Richtung relativ zur Koppelhülse 14 nach distal zu verschieben bzw. abzusenken. Der Betätigungshebel 40 weist ferner einen hier gabelförmigen Lastarm **48** auf, der mit dem Kraftarm 44 vorzugsweise einstückig verbunden ist. Der Kraftarm 44 ist mit dem Stabdrücker 24 bewegungsgekoppelt wie dies weiter unten näher erläutert ist. Der Lastarm 48 des Betätigungshebels 40 kann aus Gewichtsgründen Materialaussparungen aufweisen.

Das Griffteil 26 weist ferner ein klappenartiges Seitenwandelement **50** auf, das am Grundkörper 38 des Griffteils 26 um eine zweite Schwenkachse **52** gelagert ist. Die zweite Schwenkachse 52 ist zur Längsachse 12 des Repositionsinstruments 10 orthogonal verlaufend angeordnet. Das Seitenwandelement 50 weist einen Betätigungsfortsatz **54** auf. Ist das Seitenwandelement 50 in seiner in den Figuren 1 bis 3 gezeigten Verschlussstellung angeordnet, d.h. befindet sich das Repositionsinstruments 10 im betriebsbereiten Zustand, steht der Betätigungsfortsatz 54 über die Außenseite **56** des Griffteils 26 in radialer Richtung hervor. Das Seitenwandelement 50 ist aus der Verschlussstellung aus dem Gehäuseteil 26 herausschwenkbar, um das Repositionsinstrument 10 zu entriegeln und nach Gebrauch für Reinigungs- oder Wartungszwecke zerlegen zu können.

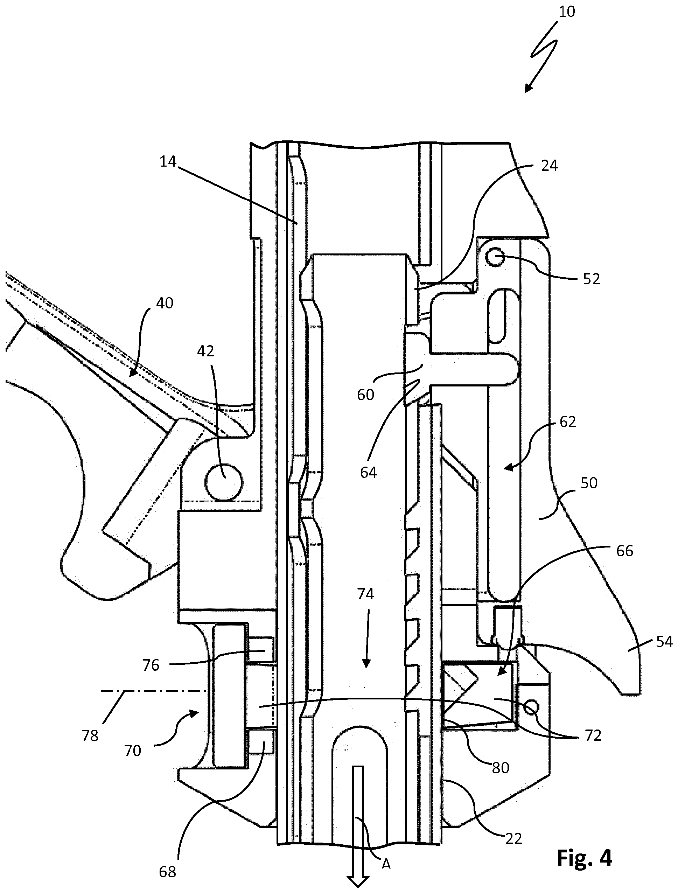

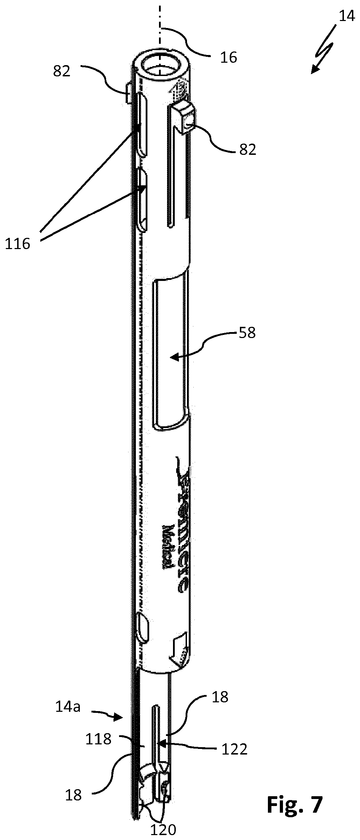

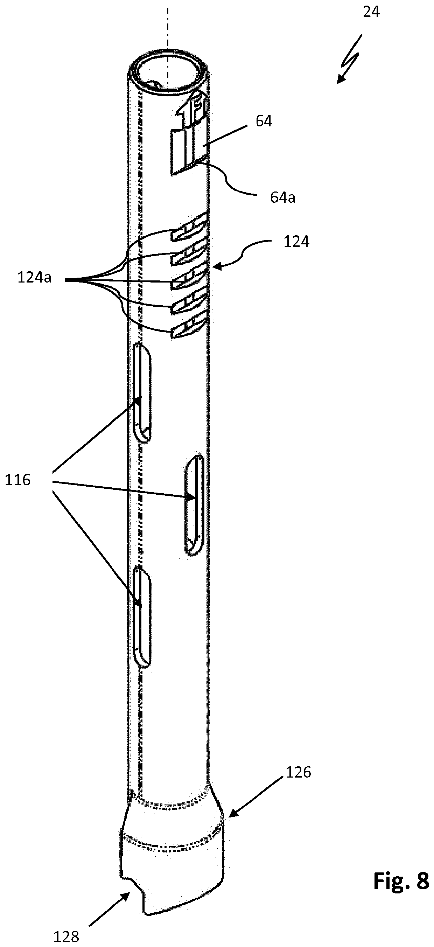

Gemäß den in den **Fign. 3** und **4** gezeigten Längsschnitten des Repositionsinstruments 10 aus Fig. 1 sowie gemäß den freigestellten Darstellungen in den Figuren 7 und 8 weisen die Arretierhülse 22 und die Koppelhülse 14 jeweils eine seitliche Wandöffnung **58** auf. Die beiden Wandöffnungen 58 sind im betriebsbereiten Zustand des Repositionsinstruments 10 in radialer Richtung zumindest abschnittsweise zueinander fluchtend angeordnet. Der gabelförmige Lastarm 48 des Betätigungshebels 40 ist mit einem Mitnehmerschlitten **60** verkoppelt. Der Mitnehmerschlitten 60 ist in einer hier T-förmigen Längsnut **62** des Seitenwandelement 50 im Gleitspiel-Formschluss längsverschieblich geführt.

Der Mitnehmerschlitten 60 erstreckt sich von außen in radialer Richtung durch die beiden seitlichen Wandöffnungen 58 der Arretierhülse 22 sowie der Koppelhülse 14 nach innen und greift in ein Mitnahmeprofil **64** des Stabdrückers 24 ein. Der Mitnehmerschlitten ist dadurch in Vorschubrichtung **A** des Stabdrückers 14 mit dem Stabdrücker 14 form- bzw. kraftschlüssig verkoppelt. Das Griffteil 26 weist weiter eine Rasteinrichtung **66** für den Stabdrücker 24 sowie auch für die Arretierhülse 22 auf. Die Rasteinrichtung 66 umfasst ein im Griffteil 26 angeordnetes Aufnahmefach **68** mit einer Seitenwandöffnung **70** sowie ein im Aufnahmefach 68 angeordnetes Rastelement **72.** Das Rastelement 72 weist eine Durchgangsöffnung **74** auf, durch die sich die Arretierhülse 22, die Koppelhülse 14 und der Stabdrücker 24 mit radialem Spiel hindurcherstrecken. Das Rastelement 72 ist mithin ringförmig ausgeformt und umgreift die Arretierhülse 22, die Koppelhülse 14 und den Stabdrücker 24 außenseitig.

Dadurch ist das Rastelement 72 im Aufnahmefach 68 zugleich gegenüber einem unerwünschten Herausfallen gesichert. Das Rastelement 72 ist bezüglich der Längsachse 12 des Repositionsinstruments 10 im Aufnahmefach mit einem nur geringen axialen Spiel angeordnet. Ein Federelement **76** dient dazu, das Rastelement 72 in Richtung seiner Bewegungsachse **78** nach außen, d.h. in Richtung der Seitenwandöffnung 70 vorzuspannen. In der gezeigten Freigabestellung der Arretierhülse 22 liegt das Rastelement 72 innenumfangsseitig mit einer Rastkante **80** (Fig. 4) an der Arretierhülse 22 außenseitig vorgespannt an. Das Rastelement 72 befindet sich hier mithin in seinem inaktiven Zustand.

Die Koppelhülse 14 weist gemäß den Fign. 3 und 7 im Bereich ihres proximalen Endabschnitts zwei federelastisch angelenkte Radialvorsprünge **82** auf, die sich jeweils in radialer Richtung nach außen durch eine langlochförmige Ausnehmung **84** der Arretierhülse 22 hindurch in dazu korrespondierende Lagerausnehmungen **86** des Griffteils 26 hineinerstrecken. Mittels der Radialvorsprünge 82 sind die Arretierhülse 22 und die Koppelhülse 14 drehfest miteinander verkoppelt und zusätzlich die Koppelhülse 14 in axialer Richtung sowie um Ihre Längsachse 16 am Griffteil 26 lagefixiert gehalten angeordnet.

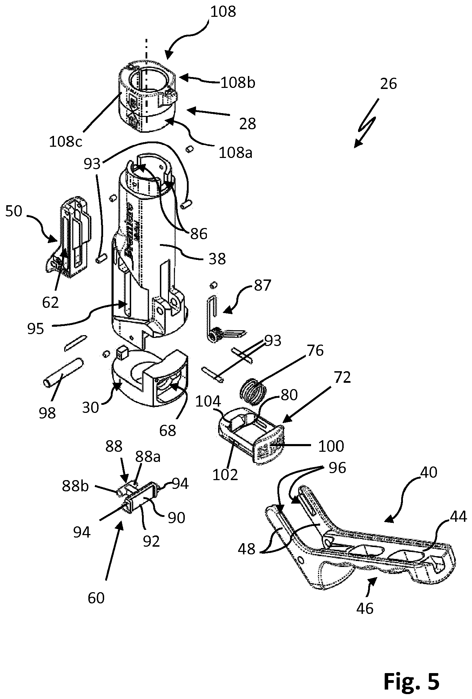

In **Fig. 5** ist das Griffteil 26 des Repositionsinstruments 10 in einer freigestellten Ansicht und mit einer explodierten Darstellung seiner Teile gezeigt.

Der Fußabschnitt 30 des Griffteils 26 kann als ein zum Grundkörper 38 des Griffteils 26 separates Bauelement ausgeführt sein und insbesondere aus Metall bestehen. Der Fußabschnitt 30 weist hier das Aufnahmefach für das Restelement 72 auf. Der Fußabschnitt 30 kann am Grundkörper 38 des Griffteils 26 durch Stifte oder dergleichen dauerhaft befestigt sein. Andere Arten der Befestigung sind natürlich vorstellbar.

Der Betätigungshebel 40 ist aus seiner in den Figuren 1 bis 3 gezeigten Ruhestellung gegen die Kraft eines vorzugsweise zweiarmigen (Torsions-) Federelements **87** auslenkbar.

Der Mitnehmerschlitten 60 ist hier beispielhaft manschettenknopfartig ausgeformt. Der Mitnehmerschlitten 60 umfasst einen T-förmigen Profilfortsatz **88** mit zwei seitlichen Vorsprüngen **88a, 88b** die im betriebsbereiten Zustand des Repositionsinstruments 10 in die T-förmige Längsnut 62 des Seitenwandelements 50 des Griffteils 26 eingreifen. Der Profilfortsatz 88 dient somit als ein Nutenstein, der in radialer Richtung zugfest in der Längsnut 62 des Seitenwandelements 50 gehalten angeordnet ist. Der Mittnehmerschlitten 60 weist eine hier plan ausgeführte Anlagefläche **90** zur radialen Anlage am Mitnahmeprofil 64 des Stabdrückers 24 auf. Eine mit **92** bezeichnete axiale Druckfläche des Mitnehmerschlittens 60 wirkt mit der Profilkante 64a des Mitnahmeprofils 64 des Stabdrückers 24 (vgl. Fig. 8) in axialer Richtung zusammen. Das Seitenwandelement 50 kann beispielsweise durch Stifte **93** am Grundkörper 38 des Griffteils 26 schwenkbar gelagert sein.

Am Mitnehmerschlitten 60 sind dabei Lagerzapfen **94** angeformt, die sich vom Mitnehmerschlitten 60 seitlich wegerstrecken. Der Mitnehmerschlitten 60 erstreckt sich in seinem Einbauzustand im Griffteil 26 in einander gegenüberliegend angeordnete seitliche Führungsschlitze **95** des Griffteils 26 hinein. Diese beiden Lagerzapfen 94 greifen im betriebsbereiten Zustand in dazu korrespondierende Spalten oder Langlöcher **96** des gabelförmigen Lastarms 48 des Betätigungshebels 40 ein. Ein mit **98** bezeichnetes Achsstück dient der schwenkbaren Lagerung des Betätigungshebels am Grundkörper 38 des Griffteils 26.

Das Rastelement 72 umfasst eine Fußplatte **100,** die über zwei Profilschenkel **102** mit einem Rastkopf **104** verbunden ist. Das Rastelement 72 weist mithin eine Steigbügelform auf. Am Rastkopf 104 ist die Rastkante 80 ausgebildet. Das Rastelement 72 besteht hier aus Metall, um eine hinreichend großes mechanisches Lastaufnahmevermögen des Rastelements 72 zu gewährleisten. Die lichte Weite der Durchgangsöffnung 74 des Rastelements 72 ist größer gewählt, als ein Außendurchmesser (nicht bezeichnet) der Arretierhülse (Fig. 3).

Der Kopfabschnitt 28 des Griffteils 26 umfasst eine ringförmige Lagerschelle **108,** die vorzugsweise aus Metall besteht. Die Lagerschelle 108 weist einen ringförmig geschlossenen ersten Längsabschnitt **108a** und einen öffenbaren zweiten Längsabschnitt **108b** auf. Der erste Längsabschnitt 108a umfasst teilweise die Lagerausnehmungen 86 für die Radialvorsprünge 82 der Koppelhülse 14 (Fig. 3). Der zweite Längsabschnitt 108b weist hier beispielhaft einen schwenkbar angelenkten Verschlussbügel **108c** auf. Der Verschlussbügel 108c dient dazu, die nach proximal offenen Lagerausnehmungen 84 des Griffteils 26 zu verschließen. Dadurch können die Koppelhülse 14 und die Arretierhülse 22 (Fig. 3) im Griffteil 26 gegenüber einem unbeabsichtigten axialen Herausbewegen dieser Bauteile aus dem Griffteil 26 nochmals besser gesichert werden.

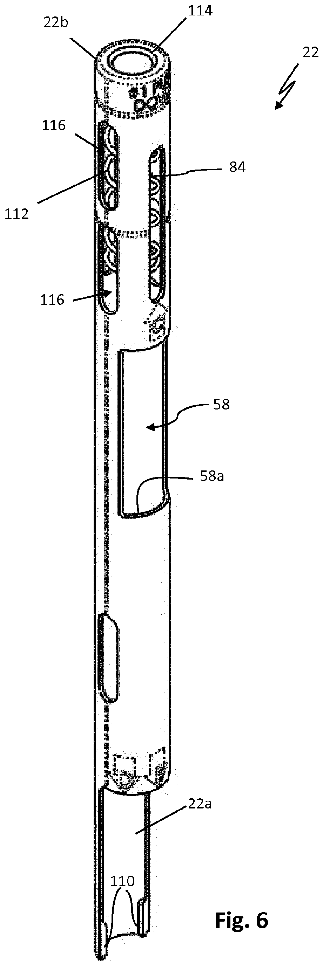

In **Fig. 6** ist die Arretierhülse 22 des Repositionsinstruments in einer freigestellten Ansicht gezeigt. Die Wandöffnung 58 für den Mitnehmerschlitten 72 des Betätigungshebels 40 (Fig. 5) ist gut zu erkennen. Ein unterer Rand der Wandöffnung 58 ist mit **58a** bezeichnet. Die Arretierhülse 22 stützt sich in ihrer Arretierstellung mit diesem unteren Rand 58a am Rastelement 76 in axialer Richtung ab. In Freigabeposition kann sich die Arretierhülse 22 über den unteren Rand 58 an dem Mitnehmerschlitten 60, bevorzugt an dessen axialer Druckfläche 92 (Fig. 5), abstützen, wie dies in Fig. 3 gezeigt ist.

Die Arretierhülse 22 weist hier nur einen Arretierfortsatz **22a** auf, der an seinem freien Randabschnitt zwei nach radial innen vorspringende bzw. aufeinander zuweisende Führungszungen **110** aufweist. Diese Führungszungen 110 erleichtern die Montage der Arretierhülse 22 auf der Koppelhülse 14 und dienen dazu, ein Abrutschen der Rastarme 18 der Koppelhülse 14 nach radial außen zu verhindern, wenn die Arretierhülse 22 in ihrer Arretierstellung angeordnet ist.

Innerhalb der Arretierhülse 22 ist ein Federelement **112** angeordnet, das sich an einem Ringbund **114** am proximalen Ende **22b** der Arretierhülse 22 in axialer Richtung abstützt.

Materialaussparungen **116** dienen einer vereinfachten Reinigung bzw. Desinfektion der Arretierhülse 22.

**Fig. 7** zeigt die Koppelhülse 14 in einer freigestellten Ansicht. Die Wandöffnung 58 weist eine zur Wandöffnung (Fig. 6) der Arretierhülse 22 entsprechende bzw. im Wesentlichen entsprechende Form und Größe auf. Die beiden flexibel angelenkten Radialvorsprünge 82 sind an einander gegenüberliegenden Bereichen der Koppelhülse 14 angeordnet. Die Koppelhülse 14 weist in einer zum Arretierfortsatz 22a der Arretierhülse 22 (Fig. 6) entsprechenden Weise einen axialen Koppelfortsatz **14a** mit den beiden Rastarmen 18 und mit einem zwischen den beiden Rastarmen 18 angeordneten Stütz- oder Führungsarm **118** auf.

Die Rastarme 18 weisen an ihrem freien Endabschnitt jeweils ein nach innen vorspringendes Koppel- oder Rastglied **120** auf, das zum Eingriff in eine dazu korrespondierende Vertiefung des Tulpenkopfs vorgesehen ist. Der Führungsarm 118 und die beiden Rastarme 18 sind voneinander jeweils durch einen Axialspalt **122** getrennt. Der Führungsarm 118 ist im Vergleich zu den Rastarmen 18 in sich biegesteif und kann im normalen Betriebseinsatz des Repositionsinstruments nicht in radialer Richtung gegenüber der Längsachse 12 ausgelenkt werden. Der Führungsarm 118 ist somit in radialer Richtung insgesamt formstabil.

**Fig. 8** zeigt den hülsenförmigen Stabdrücker 24 in einer freigestellten Ansicht. Das Mitnahmeprofil 64 für den Mitnehmerschlitten 60 (Fig. 5) ist hier als eine einzelne außenseitige Vertiefung des Stabdrückers 24 ausgeführt. Die dadurch gebildete untere Schulter oder Profilkante **64a** dient der axialen Anlage der Druckfläche 92 des Mitnehmerschlittens 60 (Fig. 5).

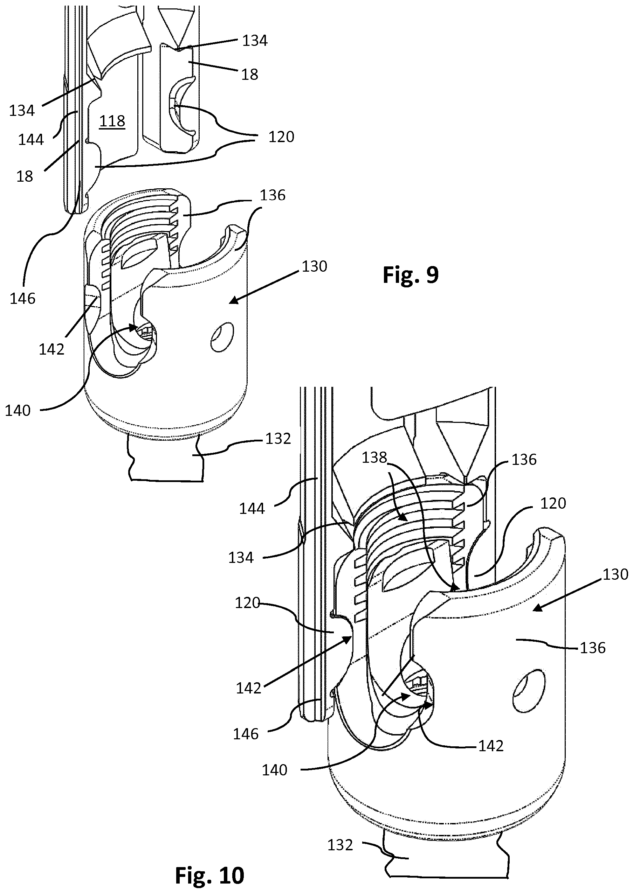

Der Stabdrücker 24 weist darüber hinaus ein außenseitiges Rastprofil **124** für den Eingriff des Rastelements 72 auf. Durch das Zusammenwirken dieser beiden Bauteile kann der Stabdrücker in seiner jeweiligen axialen Position relativ zur Koppelhülse 14 lagefixiert werden. Das Rastprofil 124 umfasst hier einzelne Nuten **124a,** die in axialer Richtung voneinander beabstandet außenseitig am Stabdrücker 24 angeordnet sind. Die Nuten 124a können gemäß Fig. 9 linear oder gekrümmt bzw. bedarfsweise als umlaufende Ringnuten ausgeführt sein. Zu beachten ist, dass der Stabdrücker 24 innenseitig glatt, d. h. stufenfrei ausgeführt ist. Der Stabdrücker 24 kann jedoch auch in axialer Richtung geriffelt bzw. wellig ausgeführt sein.

Der Stabdrücker 24 weist einen distalen Endabschnitt **126** mit stirnseitigen Vertiefungen **128** für die Aufnahme des Fixierstabs auf. Der distale Endabschnitt kann sich in axialer Richtung aufweiten. Die Materialaussparungen 116 des Stabdrückers 24 dienen im Wesentlichen der Gewichtsreduktion und vereinfachten hygienischen Aufbereitung, d. h. Reinigung und Desinfektion bzw. Sterilisation, des Repositionsinstruments.

**Fig. 9** zeigt einen vergrößerten Detailausschnitt des distalen Endabschnitts der Koppelhülse 14 beim Ankoppeln am Tulpenkopf **130** einer nur ausschnittsweise gezeigten Knochenschraube **132.** Die Rastarme 18 und der Führungsarm 118 weisen jeweils einen Axialanschlag **134** für einen Wandschenkel **136** des Tulpenkopfs 130 auf, um eine maximale axiale Einführtiefe des Tulpenkopfs 130 zwischen die Rastarme 18 zu begrenzen. Die beiden Wandschenkel 136 des Tulpenkopfs 130 sind in an sich bekannter Weise mit einem Innengwinde **138** versehen, in das eine Fixierschraube (nicht gezeigt) zur dauerhaften Festlegen des Fixierstabs (nicht gezeigt) im Tulpenkopf 130 eingeschraubt werden kann. Eine Fixierstabaufnahme des Tulpenkopfs ist mit **140** und zu den Rastgliedern 120 der Koppelhülse 14 korrespondierenden Vertiefungen sind mit **142** bezeichnet.

Zu beachten ist, dass die Rastarme 18 der Koppelhülse 14 jeweils an ihrer vom Führungsarm wegweisenden Seitenflanke **144** jeweils mit einer Längsnut **146** versehen sind. Die Längsnuten 144 setzen sich bis zum proximalen Ende der Koppelhülse 14 fort und dienen der Führung bzw. Aufnahme der Führungszungen 110 (Fig. 6) der Arretierhülse 22 beim Zusammenbau als auch während des Betriebseinsatzes des Repositionsinstruments 10.

Zum Ankoppeln des Repositionsinstruments 10 am Tulpenkopf wird die Koppelhülse 14 in axialer Richtung stirnseitig auf den Tulpenkopf 130 aufgeschoben. Die Rastarme 18 werden dabei jeweils durch deren Kontakt mit einem der Wandschenkel 136 des Tulpenkopfs 130 nach radial außen ausgelenkt, bis der Wandschenkel 136 an den Axialanschlägen 134 anschlägt und die Rastglieder 120 der Rastarme 18 in radialer Richtung in die dazu korrespondierenden außenseitigen Vertiefungen 142 des Tulpenkopfs 130 eingreifen, wie dies in **Fig. 10** verdeutlicht ist.

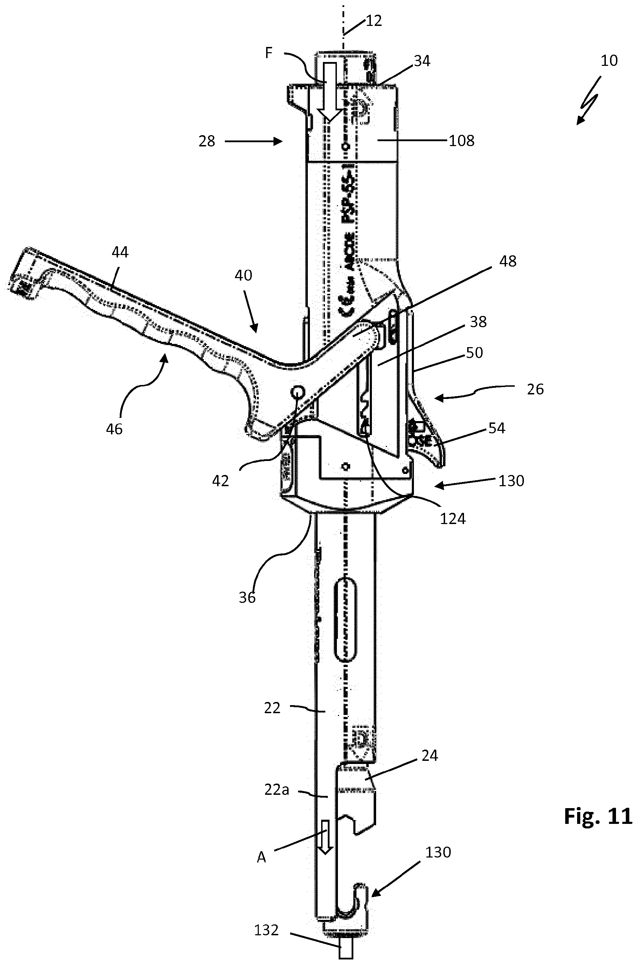

Die Koppelhülse 14 wird nachfolgend gemäß den Darstellungen in den **Figuren 11** bis **14** durch alleiniges Verschieben der Arretierhülse 22 in ihre Arretierposition am Tulpenkopf 130 der Knochenschraube 132 gesichert. Dazu wird die Arretierhülse 22 durch direkte Beaufschlagung ihres proximalen Endes 22b mit einer in Fig. 11 dargestellten axial gerichteten Kraft F manuell in ihre Arretierposition verschoben. Mit anderen Worten wird die Arretierhülse 22 durch direkten Druck eines Fingers oder mehrerer auf der Arretierhülse 22 aufgelegter Finger bzw. der aufgelegten Hand des Benutzers nach distal in seine Arretierposition verschoben. Dies erlaubt eine besonders ergonomische, gefühlvolle, kontrollierte sowie handliche Betätigung des Repositionsinstruments 10.

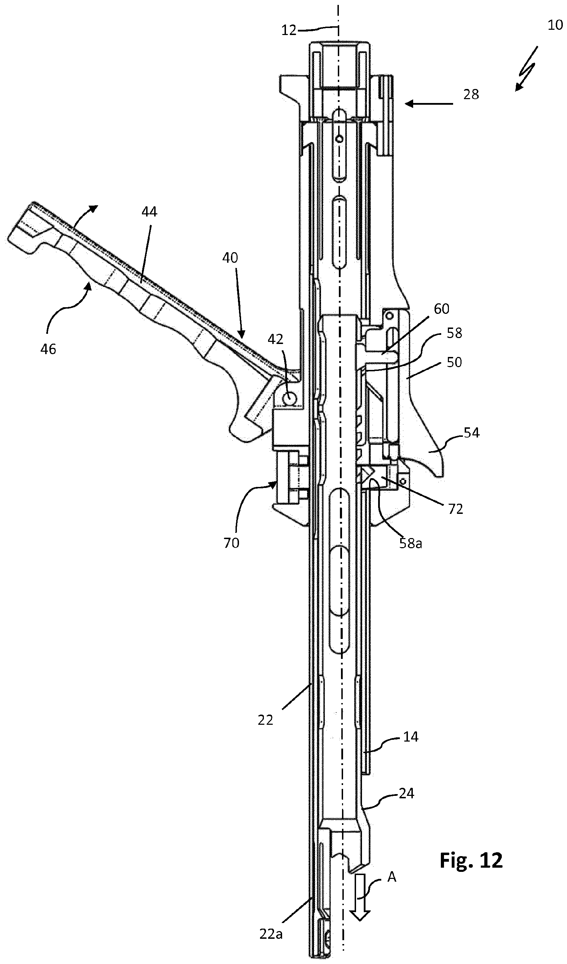

Gemäß Fig. 12 überdeckt der in Arretierposition angeordnete Arretierfortsatz 22a der Arretierhülse 22 den Koppelfortsatz 14a der Koppelhülse 14 in einer zur Längsachse 12 radialen Richtung. Ein radiales Auslenken der am Tulpenkopf verrasteten Rastarme 18 sowie ein damit einhergehendes unerwünschtes Diskonnektieren der Koppelhülse 14 vom Tulpenkopf 130 wird dadurch zuverlässig verhindert. Dadurch, dass der Arretierfortsatz 14a der Arretierhülse 22 die beiden Rastarme 18 zusätzlich im Bereich der äußeren Seitenflanken 144 (vgl. Fign. 9 und 10) übergreift und in deren jeweilige Längsnut 146 eingreift, kann einem Abrutschen der Rastarme 18 vom Tulpenkopf 130 selbst unter Last bzw. bei Angriff eines Moments entgegengewirkt werden.

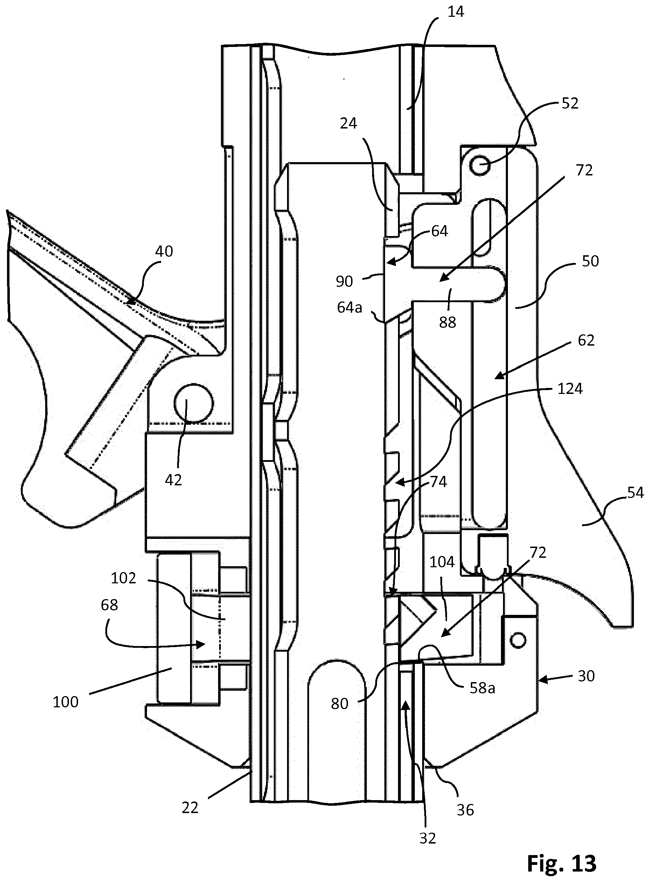

In der Arretierposition der Arretierhülse 22 ist die seitliche Wandöffnung 58 der Arretierhülse 22 zum Rastelement in radialer Richtung fluchtend angeordnet. Das Rastelement 72 wird durch die Kraft des Federelements 76 in radialer Richtung in die Arretierhülse 22 hineinbewegt, bis diese mit der Rastkante 80 am Stabdrücker 24 außenseitig unmittelbar anliegt. Dadurch ist das Rastelement 72 in seiner Rastfunktion bezüglich des Stabdrückers 24 aktiviert bzw. scharfgestellt, wie dies in Fig. 13 gezeigt ist.

Zu beachten ist, dass das Rastelement 72 hier eine Doppelfunktion aufweist. Das Rastelements 72 liegt gemäß den Fign. 12 und 13 am distalen Rand **58a** der Wandöffnung 58 der Arretierhülse 22 in axialer Richtung an. Dadurch wird ein unbeabsichtigtes axiales Zurückschieben der Arretierhülse 22 aus ihrer Arretierposition in Richtung ihrer Ruheposition zuverlässig blockiert. Das Repositionsinstrument 10 ist mithin in seiner am Kopfgehäuse 130 angekoppelten Position gesichert.

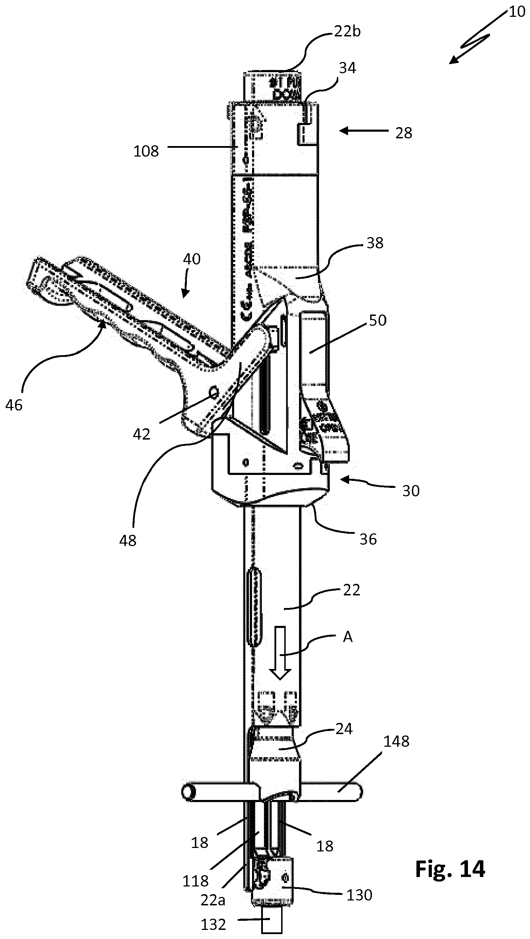

Der Fixierstab **148** kann nunmehr gemäß Fig. 14 vom Benutzer zwischen den Stabdrücker 24 und das Kopfgehäuse 130 der Knochenschraube gefädelt und nahfolgend mit dem Stabdrücker 24 in das Kopfgehäuse 130 in seine vorgegebene Endposition abgesenkt werden. Dies erfolgt mittels des Betätigungshebels 40, der bequem mit der das Griffteil haltenden Hand des Benutzers betätigt werden kann. Wird der Kraftarm 44 des Betätigungshebels 40 an das Griffteil 26 herangeschwenkt, so werden der Mitnehmerschlitten 60 und damit der Stabdrücker 24 in Vorschubrichtung A bewegt.

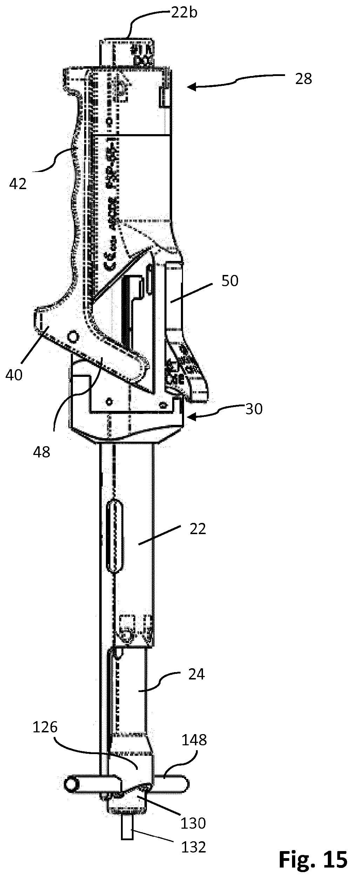

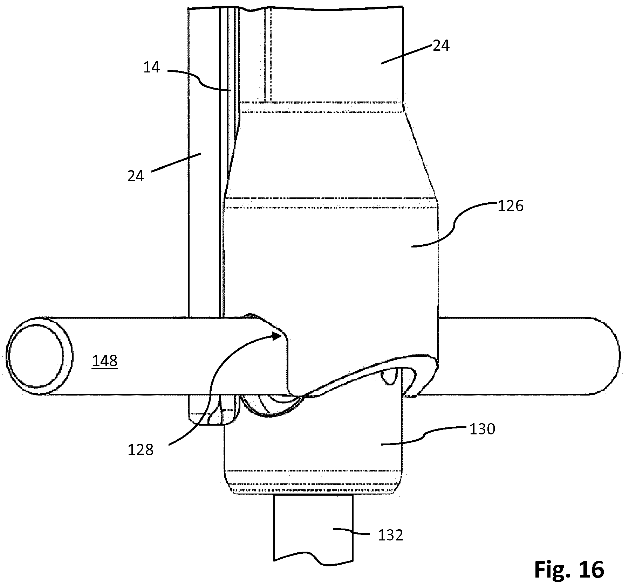

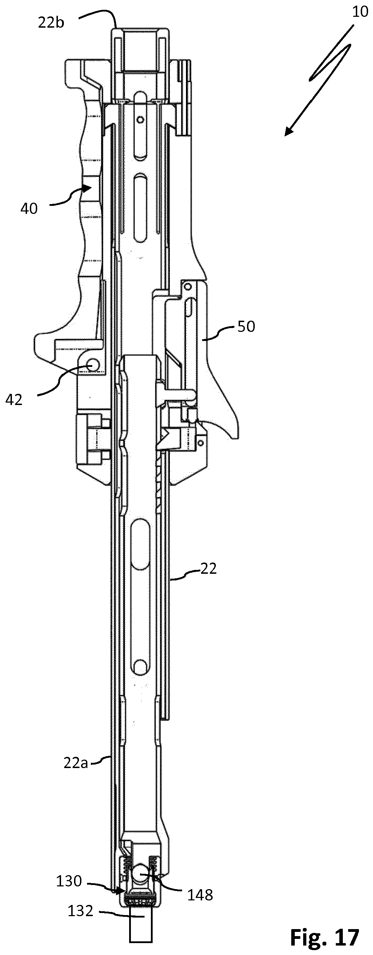

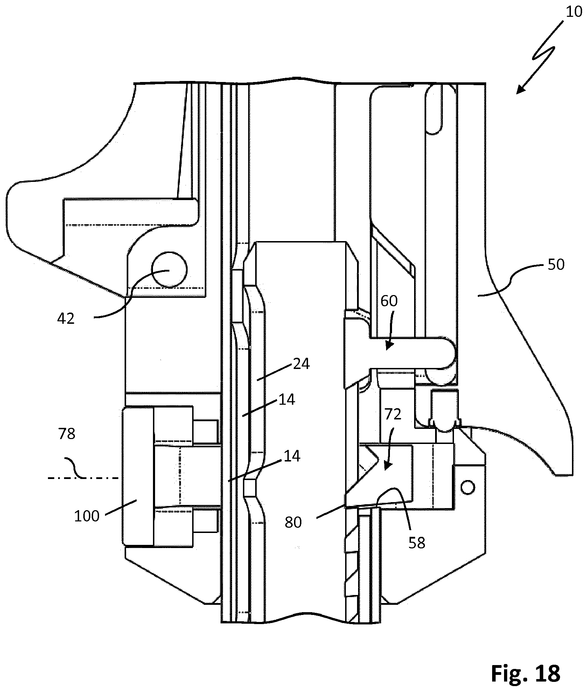

In den **Fign. 15** bis **18** ist der Fixierstab 148 vollständig in die Fixierstabaufnahme 140 (Fig. 10) des Tulpenkopfs 130 abgesenkt. Der optional erweiterte distale Endabschnitt 126 des Stabdrückers kann einen der Wandschenkel 126 des Tulpenkopfs 130 in radialer Richtung übergreifen. Der Tulpenkopf 130 ist dadurch in radialer Richtung zwischen dem Arretierfortsatz 22a und dem Koppelfortsatz 14b des Repositionsinstruments 10 gehalten angeordnet. Zur Befestigung des Fixierstabs 140 wird in an sich bekannter Weise eine Fixierschraube (nicht gezeigt) eingesetzt, die durch den hülsenartigen Stabdrücker 24 hindurch in den Tulpenkopf 130 eingebracht und mittels eines in den Stabdrücker einführbaren Drehwerkzeugs, beispielsweise eines Schraubendrehers, mit dessen Wandschenkeln 136 verschraubt wird.

Zum erneuten Lösen des Repositionsinstruments 10 vom Tulpenkopf wird das Rastelement 72 manuell in radialer Richtung, d.h. entlang der Bewegungsachse 78 des Rastelements 70 (Fig.4), weiter in das Aufnahmefach 68 des Griffteils 26 hineingedrückt. Dadurch wird das Rastelement 72 in radialer Richtung aus der Arretierhülse 22 und aus dem Rastprofil 124 des Stabdrückers 24 ausgerückt. Das Arretierhülse 22 schnappt durch die Kraft des Federelements 112 (Fig. 6) in axialer Richtung nach proximal in seine Ruheposition (Fig. 1) zurück.

Der Stabdrücker 24 gleitet durch die Kraft des (Torsions-)Federelements 87 (Fig. 5) in seine in seine in Figur 1 gezeigte Ausgangsstellung in axialer Richtung nach proximal zurück. Das Repositionsinstrument 10 steht nun für den erneuten Betriebseinsatz zur Verfügung.

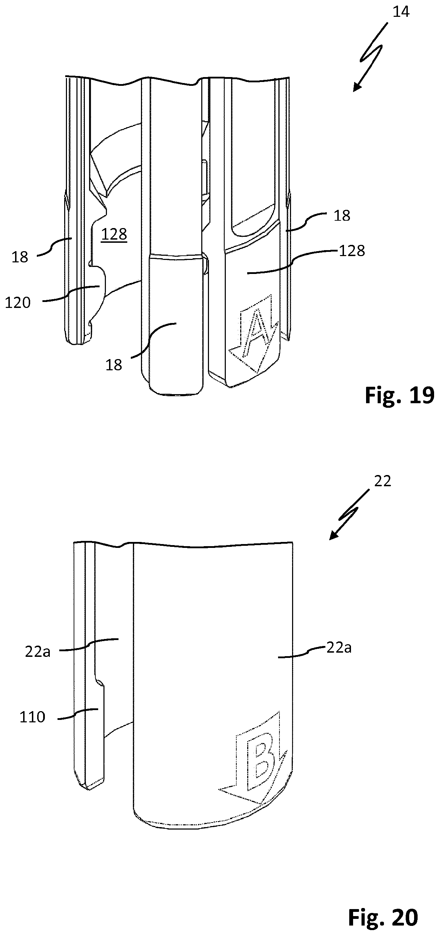

Das Repositionsinstrument 10 kann nach einer alternativen Ausführungsform auch eine Koppelhülse 14 mit insgesamt vier Rastarmen 18 und zwei Führungsarmen 118 aufweisen, wie dies in **Fig. 19** gezeigt ist. Die Rastarme 18 sind hier paarweise mit jeweils einem dazwischenliegend angeordneten Führungsarm 118 an der Koppelhülse 14 angeordnet. Wird die Koppelhülse 14 am Tulpengehäuse 130 einer Knochenschraube 132 angekoppelt, so umgreift die Koppelhülse 14 bei dieser Ausführungsform beide Wandschenkel 136 des Tulpengehäuses 130 außenseitig. Die Arretierhülse 22 weist bei dieser Bauart des Repositionsinstruments gemäß **Fig. 20** zwei Arretierfortsätze 22a auf, die in radialer Richtung einander gegenüberliegend an der Koppelhülse 22 angeordnet sind.

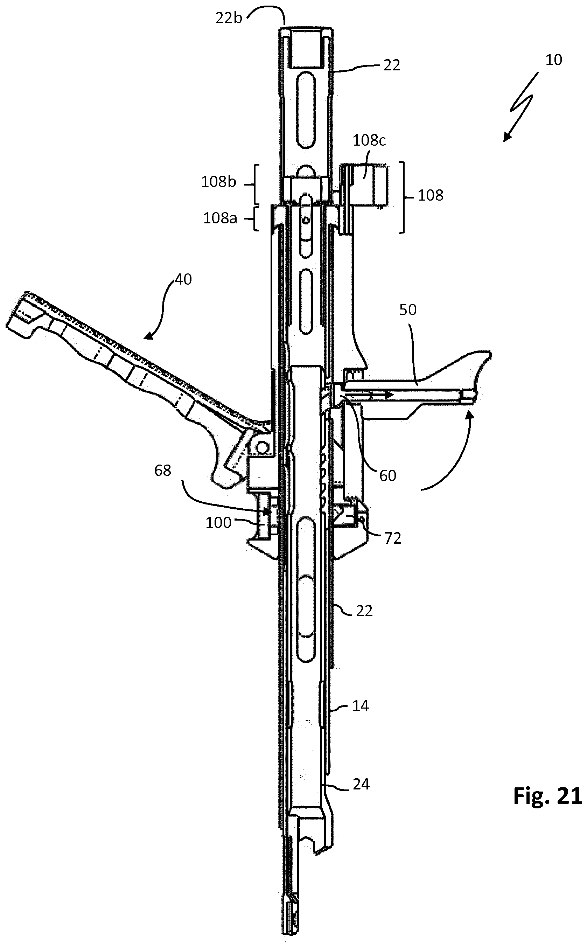

Zum Zerlegen des Repositionsinstruments 10 wird der Seitenwandabschnitt 50 in radialer Richtung aus dem Griffteil 26 herausgeschwenkt, wie dies in **Fig. 21** gezeigt ist. Dadurch wird der Mitnehmerschlitten 60 in radialer Richtung aus dem Mitnahmeprofil 64 des Stabdrückers 24 herausbewegt. Der Stabdrücker 24 kann nun in axialer Richtung nach distal, d.h. nach unten, aus der Koppelhülse 14 herausgezogen werden. Nach dem Öffnen der Lagerschelle 108 und einem radial gerichteten Eindrücken der beiden Radialvorsprünge 82 (Fig.3) der Koppelhülse 14 können die Arretierhülse 22 und die Koppelhülse 14 gemeinsam aus dem Griffteil 26 in proximaler Richtung, d.h. nach oben, herausgezogen werden.

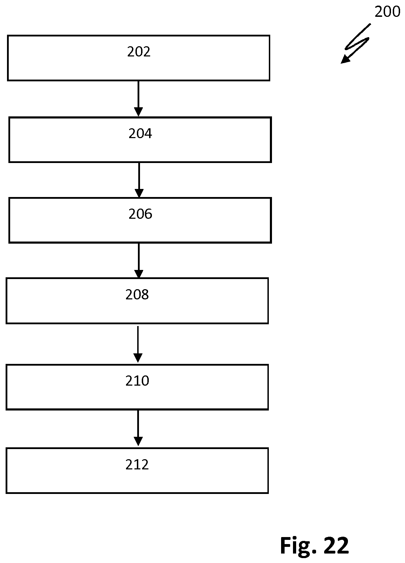

Nachfolgend wird das erfindungsgemäße Montageverfahren **200** des Repositionsinstruments 10 unter zusätzlicher Bezugnahme auf **Fig. 22** erläutert:
In einem ersten Schritt **202** werden das vormontierte Griffteil 26 sowie die Arretierhülse 22, die Koppelhülse 14 und der Stabdrücker 24 bereitgestellt.

In einem zweiten Schritt **204** wird die Arretierhülse 22 in axialer Richtung von proximal auf die Koppelhülse 14 aufgeschoben, sodass die Führungszungen 110 der Arretierhülse 22 in die dazu korrespondierenden Längsnuten 146 der Koppelhülse 14 eingreifen. Die Arretierhülse 22 wird dabei soweit auf die Koppelhülse 14 aufgeschoben, bis die Radialvorsprünge 82 der Koppelhülse 14 jeweils im zugeordneten langlochförmigen Ausnehmungen 84 der Arretierhülse 22 eingreifen bzw. einrasten.

In einem weiteren Schritt **206** werden die Arretierhülse 22 und die Koppelhülse 14 gemeinsam in axialer Richtung von proximal kommend in die Durchgangsausnehmung 32des Griffteils 26 bei geöffneter Lagerschelle 108 eingeschoben, bis die Radialvorsprünge 82 der Koppelhülse 14 in radialer Richtung in die Lagerausnehmungen 86 des Griffteils bzw. der Lagerschelle 108 eingreifen bzw. einrasten.

In einem nachfolgenden Schritt **208** wird die Lagerschelle 108 geschlossen.

Der Stabdrücker wird in einem weiteren Schritt **210** von distal kommend in axialer Richtung in die Koppelhülse 14 eingeschoben, bis dieser in seiner Ruheposition (Fig. 1) angeordnet ist.

In einem weiteren Schritt **212** wird das Seitenwandelement 50 aus seiner vom Griffteil 26 abgeschwenkten Öffnungsposition in seine Verschlussposition (Fig. 1) bewegt, so dass der Mitnehmerschlitten 60 in das Mitnahmeprofil 64 des Stabdrückers eingreift. Das Repositionsinstrument 10 steht nun für seinen Betriebseinsatz zur Verfügung.

Das vorstehend im Zusammenhang mit den Figuren 1 bis 22 erläuterte Repositionsinstrument 10 kann einen Führungsstab **150** umfassen, mit dessen Hilfe das Repositionsinstrument 10 vereinfacht an den Tulpenkopf 130 der Knochenschraube 132 herangeführt und an diesem angekoppelt werden kann. Der Führungsstab 150 weist einenends einen Kopplungsabschnitt **152** auf, der in den Tulpenkopf (Fig. 14) einführbar und festsetzbar ist. Dies kann beispielsweise über eine Schraubverbindung erfolgen. So kann der Kopplungsabschnitt mit einem Außengewinde (nicht gezeigt) versehen sein, der in das Innengewinde 138 der Wandschenkel 136 des Tulpenkopfs 130 einschraubbar ist. Bei dem in den Figuren 22A und 22B gezeigten Ausführungsbeispiel weist der Kopplungsabschnitt 152 des Führungsstabs 150 vier Kopplungsmittel **154** auf, die im Tulpenkopf 130 eingerastet bzw. verklemmt werden können. Die Kopplungsmittel 154 können durch Drücken eines anderenends am Führungsstab 150 gelagerten Betätigungsstiftes **156** in radialer Richtung nach innen bewegt werden, um den Führungsstab 150 vom Tulpenkopf 130 erneut abzukoppeln. Ist der Führungsstab 150 am Tulpenkopf 130 angekoppelt, so kann das Repositionsinstrument 10 mit dem Stabdrücker 24 auf den Führungsstab aufgefädelt und auf diesem äußerst gewebeschonend zum Tulpenkopf 130 im Operationsfeld vorgeschoben werden. Nach dem Verrasten der Koppelhülse 14 mit dem Tulpenkopf 130 wird der Führungsstab 150 vom Tulpengehäuse 130 abgekoppelt und aus dem Stabdrücker 24 nach oben herausgezogen.

## Patentansprüche

1. Chirurgisches Repositionsinstrument (10) zum Absenken und Fixieren eines Fixierstabs (148) im Tulpenkopf (130) einer Knochenschraube (132), umfassend:
- eine Koppelhülse (14) mit zumindest zwei distalen Rastarmen (18), die zum Ankoppeln des Repositionsinstruments (10) am Tulpenkopf (130) der Knochenschraube (132) in einer zur Längsachse (12) des Repositionsinstruments (10) radialen Richtung federelastisch auslenkbar sind,
- eine Arretierhülse (22), die auf der Koppelhülse (14) zwischen einer Freigabeposition und einer Arretierposition längsverschiebbar angeordnet ist, wobei die Auslenkung der Rastarme (18) durch die Arretierhülse (22) in ihrer Freigabeposition ermöglicht und in ihrer Arretierstellung gehemmt ist;
- ein Stabdrücker (24) zum Kontaktieren und Absenken des Fixierstabs (148), der innerhalb der Koppelhülse (14) verschiebbar angeordnet ist;
- ein Griffteil (26) mit einer Durchgangsausnehmung (32), in der die Arretierhülse (22), die Koppelhülse (14) und der Stabdrücker (24) angeordnet sind;
- einen am Griffteil (26) schwenkbar gelagerten Betätigungshebel (40) zum Vorschieben des Stabdrückers (24) relativ zur Koppelhülse (14), mit einem Kraftarm (44), der manuell aus seiner vom Griff (26) seitlich wegstehenden Ruhestellung an das Griffteil (26) heranbewegbar ist und mit einem Lastarm (48), der mit dem Stabdrücker verkoppelt ist,
wobei die Arretierhülse (22) in ihrer Freigabeposition mit ihrem proximalen Ende (22b) aus der Durchgangsausnehmung (32) in axialer Richtung über das Griffteil (26) hervorsteht und unabhängig vom Stabdrücker (24) durch Beaufschlagung ihres proximalen Endes (22b) mit einer axial gerichteten Kraft (F) in ihre Arretierposition verschiebbar ist.

2. Repositionsinstrument nach Anspruch 1, **dadurch gekennzeichnet, dass** die Arretierhülse in ihrer Arretierposition relativ zur Koppelhülse verrastbar ist.

3. Repositionsinstrument nach Anspruch 1, **dadurch gekennzeichnet, dass** der Lastarm (48) des Betätigungshebels (40) über einen im Griffteil (26) längsverschieblich gelagerten Mitnehmerschlitten (60) am Stabdrücker angreift.

4. Repositionsinstrument nach Anspruch 3, **dadurch gekennzeichnet, dass** der Mitnehmerschlitten (60) Lagerzapfen (94) aufweist, die jeweils in ein Langloch (96) des Betätigungshebels (40) eingreifen.

5. Repositionsinstrument nach Anspruch 3 oder 4, **dadurch gekennzeichnet, dass** das Griffteil (26) ein Seitenwandelement (50) mit einer, vorzugsweise T-förmigen, Längsnut (62) aufweist, in der der Mitnehmerschlitten (60) geführt ist.

6. Repositionsinstrument nach Anspruch 5, **dadurch gekennzeichnet, dass** das Seitenwandelement (50) am Griffteil (26) schwenkbar gelagert ist.

7. Repositionsinstrument nach einem der vorhergehenden Ansprüche, **dadurch gekennzeichnet, dass** die Arretierhülse (22) und die Koppelhülse (14) jeweils eine seitliche Wandöffnung (58) aufweisen, die im betriebsbereiten Zustand des Repositionsinstruments in radialer Richtung zumindest teilweise zueinander fluchtend angeordnet sind, wobei sich der Mitnehmerschlitten (60) durch die beiden Wandöffnungen (58) in radialer Richtung hindurcherstreckt.

8. Repositionsinstrument nach einem der vorhergehenden Ansprüche, **dadurch gekennzeichnet, dass** der Betätigungshebel (40) gegen die Kraft eines Federelements (76), insbesondere eines Torsionsfederelements, aus seiner Ruhestellung herausbewegbar ist.

9. Repositionsinstrument nach einem der vorhergehenden Ansprüche, **dadurch gekennzeichnet, dass** die Koppelhülse (14) zwei, vorzugsweise federelastisch angelenkte, Radialvorsprünge (82) aufweist, die sich jeweils in radialer Richtung durch eine langlochförmige Ausnehmung (84) der Arretierhülse (22) hindurch in eine Lagerausnehmung (86) des Griffteils (26) hineinerstrecken.

10. Repositionsinstrument nach einem der vorhergehenden Ansprüche, **dadurch gekennzeichnet, dass** das Griffteil (26) einenends eine öffenbare Lagerschelle (108) aufweist, die vorzugsweise aus Metall besteht und die vorzugsweise die Lagerausnehmungen (86) für die Arretierhülse (22) aufweist.

11. Repositionsinstrument nach einem der vorhergehenden Ansprüche, **dadurch gekennzeichnet, dass** der Stabdrücker (24) mittels einer Rasteinrichtung (66) in seiner jeweiligen axialen Position relativ zur Koppelhülse (14) verrastbar ist.

12. Repositionsinstrument nach Anspruch 11, **dadurch gekennzeichnet, dass** die Rasteinrichtung (66) ein Rastelement (72) umfasst, das im Griffteil (26), vorzugsweise verschiebbar, gelagert ist und das mit einem Rastprofil (124) des Stabdrückers (24) zusammenwirkt.

13. Repositionsinstrument nach Anspruch 12, **dadurch gekennzeichnet, dass** das Rastelement (72) die Arretierhülse (22), die Koppelhülse (14) und den Stabdrücker (24) ringförmig umgreift.

14. Repositionsinstrument nach Anspruch 12, **dadurch gekennzeichnet, dass** das Rastelement (72) durch die Kraft eines Federelements (76) radial in Richtung seiner Raststellung am Stabdrücker vorgespannt angeordnet ist.

15. Repositionsinstrument nach einem der Ansprüche 12 bis 14, **dadurch gekennzeichnet, dass** die Arretierhülse (22) mittels des Rastelements (72) in seiner Arretierposition relativ zur Koppelhülse (14) arretierbar ist.

16. Repositionsinstrument nach einem der Ansprüche 12 bis 15, **dadurch gekennzeichnet, dass** sich das Rastelement (72) in Arretierposition der Arretierhülse abschnittsweise über die Wandöffnung (58) der Arretierhülse (22) in radialer Richtung in die Arretierhülse (22) hineinerstreckt und in axialer Richtung an einem unteren Rand (58a) der Wandöffnung (58) des Arretierelements (22) anliegt.

17. Repositionsinstrument nach einem der vorhergehenden Ansprüche, **dadurch gekennzeichnet, dass** die Koppelhülse (14) an ihrem distalen freien Endabschnitt einen Führungsarm (118) aufweist, der sich in axialer Richtung zwischen den beiden Rastzungen (18) erstreckt und von diesen jeweils unter Ausbildung eines Axialspalts (122) beabstandet ist, wobei der Führungsarm (118) im Vergleich zu den Rastzungen (18) biegesteif, d.h. in radialer Richtung formstabil, ausgebildet ist.

18. Repositionsinstrument nach einem der vorhergehenden Ansprüche, **dadurch gekennzeichnet, dass** die Koppelhülse (14) zwei weitere Rastarme (18) aufweist, zwischen denen sich in axialer Richtung vorzugsweise eine weitere Anschlagszunge (128) erstreckt.

19. Repositionsinstrument nach einem der vorhergehenden Ansprüche, **dadurch gekennzeichnet, dass** die Arretierhülse (14) an ihrem distalen freien Endabschnitt Führungszungen (110) aufweist, die jeweils in eine dazu korrespondierende Längsnut (146) der Koppelhülse (14) eingreifen, wobei sich die Längsnuten (146) jeweils zumindest abschnittsweise an einer Seitenflanke (144) eines der Rastarme (18) erstrecken.

20. Repositionsinstrument nach Anspruch 19, **dadurch gekennzeichnet, dass** sich die Längsnuten (146) jeweils bis zum proximalen Ende (14b) der Koppelhülse (14) erstrecken.

21. Repositionsinstrument nach einem der vorhergehenden Ansprüche, **dadurch gekennzeichnet, dass** die Arretierhülse (22) gegen die Kraft eines, vorzugsweise innerhalb der Arretierhülse (22) angeordneten, Federelements (112) aus seiner Freigabeposition in die Arretierposition verschiebbar ist.

## Claims

1. A surgical repositioning instrument (10) for lowering and fixing a fixing rod (148) in the tulip head (130) of a bone screw (132), comprising:
- a coupling sleeve (14) having at least two distal latching arms (18) which are resiliently deflectable for coupling the repositioning instrument (10) to the tulip head (130) of the bone screw (132) in a radial direction to the longitudinal axis (12) of the repositioning instrument (10),
- a locking sleeve (22) which is longitudinally displaceably arranged on the coupling sleeve (14) between a release position and a locking position, wherein the deflection of the latching arms (18) by the locking sleeve (22) is allowed in its release position and is inhibited in its locking position;
- a rod presser (24) for contacting and lowering the fixing rod (148) slidably arranged within the coupling sleeve (14);
- a handle part (26) having a through bore (32) in which the locking sleeve (22), the coupling sleeve (14) and the rod presser (24) are arranged;
- actuating lever (40)
- pivotally mounted on the handle part (26) for advancing the presser rod (24) relative to the coupling sleeve (14),
with a force arm (44), which can be manually moved from its rest position projecting laterally from the handle (26) to the handle part (26) and
with a load arm (48) coupled to the rod presser,
wherein the locking sleeve (22) protrudes in its release position with its proximal end (22b) from the through bore (32) in the axial direction over the handle part (26) and is displaceable into its locking position independently of the rod presser (24) by acting on its proximal end (22b) with an axially directed force (F).

2. The repositioning instrument according to claim 1, **characterized in that** the locking sleeve can be latched in its locking position relative to the coupling sleeve.

3. The repositioning instrument according to claim 1, **characterized in that** the load arm (48) of the actuating lever (40) engages over a longitudinally displaceably mounted carrier slide (60) in the handle part (26) on the rod presser.

4. The repositioning instrument according to claim 3, **characterized in that** the carrier slide (60) has bearing journals (94) which engage in each case in a slot (96) of the actuating lever (40).

5. The repositioning instrument according to claim 3 or 4, **characterized in that** the handle part (26) has a side wall element (50) with a, preferably T-shaped, longitudinal groove (62) in which the carrier slide (60) is guided.

6. The repositioning instrument according to claim 5, **characterized in that** the side wall element (50) is pivotally mounted on the handle part (26).

7. The repositioning instrument according to any of the preceding claims, **characterized in that** the locking sleeve (22) and the coupling sleeve (14) each have a lateral wall opening (58) which are arranged in the operative state of the repositioning instrument in the radial direction at least partially aligned with each other, wherein the carrier slide (60) extends through the two wall openings (58) in the radial direction.

8. The repositioning instrument according to any of the preceding claims, **characterized in that** the actuating lever (40) can be moved out of its rest position against the force of a spring element (76), in particular a torsion spring element.

9. The repositioning instrument according to any of the preceding claims, **characterized in that** the coupling sleeve (14) has two, preferably resiliently articulated, radial projections (82) each extending in the radial direction through a slot-shaped recess (84) of the locking sleeve (22) into a bearing recess (86) of the handle part (26).

10. The repositioning instrument according to any of the preceding claims, **characterized in that** the handle part (26) has at one end an openable bearing bracket (108), which preferably consists of metal and which preferably has the bearing recesses (86) for the locking sleeve (22).

11. The repositioning instrument according to any of the preceding claims, **characterized in that** the rod presser (24) can be latched by means of a latching device (66) in its respective axial position relative to the coupling sleeve (14).

12. The repositioning instrument according to claim 11, **characterized in that** the latching device (66) comprises a latching element (72) which is in the grip part (26), preferably displaceably mounted, and which works together with a latching profile (124) of the rod presser (24).

13. The repositioning instrument according to claim 12, **characterized in that** the latching element (72) surrounds the locking sleeve (22), the coupling sleeve (14) and the rod presser (24) in an annular manner.

14. The repositioning instrument according to claim 11 or 12, **characterized in that** the latching element (72) is arranged radially biased by the force of a spring element (76) in the direction of its detent position on the rod presser.

15. The repositioning instrument according to any of claims 12 to 14, **characterized in that** the locking sleeve (22) can be locked by means of the latching element (72) in its locking position relative to the coupling sleeve (14).

16. The repositioning instrument according to any of the preceding claims, **characterized in that** the latching element (72) in the locking position of the locking sleeve extends in sections over the wall opening (58) of the locking sleeve (22) in the radial direction into the locking sleeve (22) and abuts in the axial direction a lower edge (58a) of the wall opening (58) of the locking element (22).

17. The repositioning instrument according to any of the preceding claims, **characterized in that** the coupling sleeve (14) has at its distal free end portion a guide arm (118) which extends in the axial direction between the two locking tongues (18) and is spaced apart from each of these to form an axial gap (122), wherein the guide arm (118) is rigid in comparison to the locking tongues (18), i.e. is formed dimensionally stably in the radial direction.

18. The repositioning instrument according to any of the preceding claims, **characterized in that** the coupling sleeve (14) has two further latching arms (18), between which preferably a further stop tongue (128) extends in the axial direction.

19. The repositioning instrument according to any of the preceding claims, **characterized in that** the locking sleeve (14) at its distal free end portion has guide tongues (110), each engaging in a corresponding longitudinal groove (146) of the coupling sleeve (14), wherein the longitudinal grooves (146) each extend at least in sections on a side edge (144) of one of the latching arms (18).

20. The repositioning instrument according to claim 19, **characterized in that** the longitudinal grooves (146) each extend to the proximal end (14b) of the coupling sleeve (14).

21. The repositioning instrument accordin to any of the preceding claims, **characterized in that** the locking sleeve (22) can be moved against the force of a spring element (112), preferably arranged within the locking sleeve (22), from its release position into the locking position.

## Revendications

1. Instrument chirurgical (10) de repositionnement, dévolu à l'abaissement et au blocage à demeure d'une barrette d'assujettissement (148) dans la tête (130) de la tulipe d'une vis d'ostéosynthèse (132), comprenant :
- une douille de couplage (14) munie d'au moins deux bras encliquetables distaux (18) qui peuvent être animés élastiquement d'une excursion pivotante, dans une direction radiale par rapport à l'axe longitudinal (12) de l'instrument de repositionnement (10), en vue du couplage dudit instrument de repositionnement (10) avec la tête (130) de la tulipe de ladite vis d'ostéosynthèse (132) ;
- un manchon d'arrêt (22) agencé à coulissement longitudinal entre un emplacement de libération et un emplacement d'arrêt, sur ladite douille de couplage (14), l'excursion pivotante des bras encliquetables (18) étant autorisée lorsque ledit manchon d'arrêt (22) occupe son emplacement de libération, et entravée lorsqu'il occupe sa position d'arrêt ;
- un poussoir (24) logé à coulissement à l'intérieur de la douille de couplage (14), conçu pour entrer en contact avec la barrette d'assujettissement (148) et pour abaisser cette dernière ;
- une partie de préhension (26) pourvue d'un évidement de passage (32) dans lequel le manchon d'arrêt (22), la douille de couplage (14) et le poussoir (24) de barrette sont intégrés ;
- un levier d'actionnement (40) monté à pivotement sur la partie de préhension (26), conçu pour faire avancer ledit poussoir (24) de barrette par rapport à ladite douille de couplage (14), et doté d'un bras de force (44) qui peut être rapproché manuellement de ladite partie de préhension (26), à partir de sa position de repos dépassant latéralement au-delà de la poignée (26) ; et
- un bras de charge (48) couplé avec ledit poussoir de barrette,
sachant que le manchon d'arrêt (22) occupant son emplacement de libération dépasse de l'évidement de passage (32) dans la direction axiale par son extrémité proximale (22b), au-delà de la partie de préhension (26), et peut être amené à son emplacement d'arrêt, indépendamment du poussoir (24) de barrette, sous l'effet d'une sollicitation de son extrémité proximale (22b) par une force (F) dirigée axialement.

2. Instrument de repositionnement selon la revendication 1, **caractérisé par le fait que** le manchon d'arrêt occupant son emplacement d'arrêt peut être encliqueté par rapport à la douille de couplage.

3. Instrument de repositionnement selon la revendication 1, **caractérisé par le fait que** le bras de charge (28) du levier d'actionnement (40) vient en prise avec le poussoir de barrette par l'intermédiaire d'un curseur d'entraînement (60) monté à coulissement longitudinal dans la partie de préhension (26).

4. Instrument de repositionnement selon la revendication 3, **caractérisé par le fait que** le curseur d'entraînement (60) comporte des tourillons (94) pénétrant, respectivement, dans un trou oblong (96) du levier d'actionnement (40).

5. Instrument de repositionnement selon la revendication 3 ou 4, **caractérisé par le fait que** la partie de préhension (26) comporte un élément de paroi latérale (50) muni d'une rainure longitudinale (62), de préférence configurée en T, dans laquelle le curseur d'entraînement (60) est guidé.

6. Instrument de repositionnement selon la revendication 5, **caractérisé par le fait que** l'élément de paroi latérale (50) est monté à pivotement sur la partie de préhension (26).

7. Instrument de repositionnement selon l'une des revendications précédentes, **caractérisé par le fait que** le manchon d'arrêt (22) et la douille de couplage (14) sont pourvus d'ouvertures latérales respectives (58) pratiquées dans les parois et disposées, au moins en partie, avec alignement mutuel dans la direction radiale lorsque ledit instrument de repositionnement est à l'état d'aptitude au fonctionnement, le curseur d'entraînement (60) traversant de part en part, dans la direction radiale, les deux ouvertures (58) pratiquées dans les parois.

8. Instrument de repositionnement selon l'une des revendications précédentes, **caractérisé par le fait que** le levier d'actionnement (40) peut être déplacé hors de sa position de repos en opposition à la force d'un élément élastique (76), en particulier d'un élément à ressort de torsion.

9. Instrument de repositionnement selon l'une des revendications précédentes, **caractérisé par le fait que** la douille de couplage (14) est munie de deux protubérances radiales (82) préférentiellement articulées de manière élastique, qui traversent à chaque fois de part en part, dans la direction radiale, un évidement (84) en forme de trou oblong pratiqué dans le manchon d'arrêt (22), et pénètrent dans un évidement de support (86) de la partie de préhension (26).

10. Instrument de repositionnement selon l'une des revendications précédentes, **caractérisé par le fait que** la partie de préhension (26) est dotée, à une extrémité, d'une bride de montage (108) apte à l'ouverture, préférentiellement constituée d'un métal et comportant, de préférence, les évidements de support (86) destinés au manchon d'arrêt (22).

11. Instrument de repositionnement selon l'une des revendications précédentes, **caractérisé par le fait que** le poussoir (24) de barrette peut être cranté, au moyen d'un dispositif d'encliquetage (66), à l'emplacement axial qu'il occupe respectivement par rapport à la douille de couplage (14).

12. Instrument de repositionnement selon la revendication 11, **caractérisé par le fait que** le dispositif d'encliquetage (66) inclut un élément encliquetable (72) monté, de préférence, à coulissement dans la partie de préhension (26) et coopérant avec un profil de crantage (124) du poussoir (24) de barrette.

13. Instrument de repositionnement selon la revendication 12, **caractérisé par le fait que** l'élément encliquetable (72) ceinture annulairement le manchon d'arrêt (22), la douille de couplage (14) et le poussoir (24) de barrette.

14. Instrument de repositionnement selon la revendication 12, **caractérisé par le fait que** l'élément encliquetable (72) est agencé avec précontrainte radiale, par la force d'un élément élastique (76), dans la direction de sa position encliquetée sur le poussoir de barrette.

15. Instrument de repositionnement selon l'une des revendications 12 à 14, **caractérisé par le fait que** le manchon d'arrêt (22) peut être bloqué, au moyen de l'élément encliquetable (72), dans son emplacement d'arrêt par rapport à la douille de couplage (14).

16. Instrument de repositionnement selon l'une des revendications 12 à 15, **caractérisé par le fait qu'**à l'emplacement d'arrêt du manchon d'arrêt, l'élément encliquetable (72) pénètre radialement par zones dans ledit manchon d'arrêt (22), par l'intermédiaire de l'ouverture (58) pratiquée dans la paroi dudit manchon d'arrêt (22), et est axialement en applique contre un bord inférieur (58a) de ladite ouverture (58) pratiquée dans la paroi de l'élément d'arrêt (22).

17. Instrument de repositionnement selon l'une des revendications précédentes, **caractérisé par le fait que** la douille de couplage (14) est munie, au niveau de sa région extrême distale libre, d'un bras de guidage (118) qui s'étend, dans la direction axiale, entre les deux languettes encliquetables (18) dont il est espacé en réservant, à chaque fois, un espace interstitiel axial (122), ledit bras de guidage (118) étant réalisé avec rigidité à la flexion comparativement auxdites languettes encliquetables (18), c'est-à-dire avec stabilité de forme dans la direction radiale.

18. Instrument de repositionnement selon l'une des revendications précédentes, **caractérisé par le fait que** la douille de couplage (14) est pourvue de deux bras encliquetables supplémentaires (18) entre lesquels, de préférence, une languette additionnelle de butée (128) s'étend dans la direction axiale.

19. Instrument de repositionnement selon l'une des revendications précédentes, **caractérisé par le fait que** le manchon d'arrêt (22) est doté, au niveau de sa région extrême distale libre, de languettes de guidage (110) pénétrant, à chaque fois, dans une rainure longitudinale (146) de forme complémentaire, pratiquée dans la douille de couplage (14), les rainures longitudinales (146) s'étendant à chaque fois, au moins par zones, sur un flanc (144) de l'un des bras encliquetables (18).

20. Instrument de repositionnement selon la revendication 19, **caractérisé par le fait que** les rainures longitudinales (146) s'étendent, à chaque fois, jusqu'à l'extrémité proximale (14b) de la douille de couplage (14).

21. Instrument de repositionnement selon l'une des revendications précédentes, **caractérisé par le fait que** le manchon d'arrêt (22) peut être amené à l'emplacement d'arrêt, à partir de son emplacement de libération, en opposition à la force d'un élément élastique (112) préférentiellement logé à l'intérieur dudit manchon d'arrêt (22).
